# EUROPEAN PATENT APPLICATION

(11) **EP 4 603 151 A1**
(43) Date of publication of application: **20.08.2025**
(21) Application number: 24201443.9
(22) Date of filing: 19.09.2024
(51) Int. Cl.: A61Q 17/00, A61Q 19/00, A61Q 5/00, A61K 8/34, A61K 8/55, A61K 8/64, A61K 8/365, A61K 8/92, A61K 8/9789

(54) **STABLE COSMETIC COMPOSITION, METHODS AND USES THEREOF**

(30) Priority: 16.02.2024 PT 2024119259
(71) Applicant: Solfarcos Soluções Farmaceuticas e Cosmeticas Lda, 4700-034 Braga (PT)
(72) Inventor: PEREIRA GUIMARÃES, DIANA ISABEL, 4700-034 BRAGA (PT); SÁ LOUREIRO, ANA ISABEL, 4700-034 BRAGA (PT); CAVACO PAULO, ARTUR MANUEL, 4700-034 BRAGA (PT)
(74) Representative: Patentree

(57) **Abstract**

A stable emulsified cosmetic composition comprising a bioactive mixture, a phospholipid as an emulsifier, an oil component and at least one diol with enhanced stability, moisturizing effect, antioxidant benefits, and antimicrobial properties for application on skin, hair, nails, and/or scalp. The composition comprises a carefully selected blend of emulsifiers, antioxidants, and antimicrobial agents, ensuring the stability of the emulsion over time and promoting a suitable treatment for beauty healthcare.

## Description

### TECHNICAL FIELD

The present disclosure relates to a stable emulsified cosmetic waterless composition to enhance specific attributes of the skin, hair, nail, and/or scalp. This stable cosmetic composition has a better shell stability, moisturizing, antioxidant and antimicrobial properties.

### BACKGROUND

Cosmetic compositions often face challenges related to instability, susceptibility to oxidation, and microbial contamination, which can compromise their efficacy and safety. The present disclosure addresses these challenges by providing a stable emulsified composition with enhanced moisturizing, antioxidant and antimicrobial properties, suitable for various cosmetic applications.

These facts are disclosed in order to illustrate the technical problem addressed by the present disclosure.

### GENERAL DESCRIPTION

A stable emulsified cosmetic composition is disclosed herein, formulated to provide enhanced stability, antioxidant benefits, and antimicrobial properties for application on skin, hair, nails, and/or scalp. The composition comprises a carefully selected blend of emulsifiers, antioxidants, and antimicrobial agents, ensuring the stability of the emulsion over time and promoting skin health.

The present disclosure pertains to stable emulsified waterless composition comprising a green solvent, which can be a naturally occurring product, a mixture of them or a NAtural Deep Eutectic Solvent (NADES), serving as a solvent itself or to extract bioactive compounds, along with an emulsifier (e.g. phospholipids), an oil phase, and at least one soluble auxiliary, such as a diol. Furthermore, the composition of the present disclosure may further include a peptide, a cosmetic component, or a combination thereof, with the intent of addressing and enhancing specific attributes of hair, nail, scalp, and skin.

The stable emulsified cosmetic composition disclosed herein can be formulated into various skincare, haircare, nail care, and scalp care products, including but not limited to lotions, creams, serums, shampoos, conditioners, and treatments. Its stability, antioxidant activity, and antimicrobial efficacy make it suitable for use in a wide range of cosmetic formulations, catering to diverse consumer needs.

NADES are a type of solvent formed by mixing two or more naturally occurring compounds at a certain ratio, resulting in a eutectic mixture with unique properties. They are gaining attention as environmentally friendly alternatives to traditional solvents in various applications, including pharmaceuticals, cosmetics, and green chemistry with high efficiency in extracting natural components.

The present disclosure relates to a stable emulsified cosmetic composition that provides a synergistic blend of stability, moisturizing, antioxidant, and antimicrobial properties, thereby positioning it as a valuable component for crafting premium cosmetic products.

The present disclosure relates to a stable emulsified cosmetic composition, comprising: a bioactive mixture; a phospholipid as an emulsifier; and di-alcohol as a solvent; wherein bioactive mixture comprises a green solvent as NADES and an active cosmetic compound; wherein the NADES is a mixture of lactic acid and glycerol. Preferably for better results the composition does not comprise water.

Phospholipids (unsaturated or hydrogenated) are used as skin-friendly emulsifier agents. They help to repair and restore our skin's natural protective barrier. They mimic the lamellar structure of the extracellular matrix and form a protective layer, preventing water loss from the skin. Phospholipids themselves can be used as a powerful cosmetic active ingredient for skin protection and skin rejuvenation, and as a carrier system for cosmetic active ingredients improving their skin interaction, resulting in a long-lasting cosmetic effect.

Green solvents (e.g. NADES) as bio-solvents to dissolve cosmetic ingredients or to prepare natural extracts from plants or insects are used in cosmetic products with several antioxidant and antimicrobial activity.

Alcohols such as a diol, are versatile ingredients used as solvents and viscosity decreasing agents in cosmetic formulations. They are also used as moisturizing agents, emollients and humectants in cosmetics products.

The oil phase influences the final characteristics of a cosmetic product, including its spreading behaviour and sensory properties. It can provide also antioxidant and antimicrobial properties.

An aspect of the present disclosure relates to a stable emulsified cosmetic composition, comprising:
a bioactive mixture; a phospholipid as an emulsifier; and di-alcohol as a solvent;
wherein the bioactive mixture comprises a natural deep eutectic solvent and an active cosmetic compound;
wherein the natural deep eutectic solvent is selected from a list consisting of a mixture of at least two of: lactic acid, glycolic acid, decanoic acid, citric acid, maleic acid, malic acid, oxalic acid, tartaric acid, oleic acid, palmitic acid, enanthic acid,
formic acid, caprylic acid, ethylene glycol, propylene glycol, glycine, glycerol, glucose, transcutol, menthol, sodium lactate, sodium acetate, sodium citrate, xylitol and sorbitol more preferably lactic acid, glycolic acid, glycine, glycerol, ethylene glycol and propylene glycol.

The cosmetic composition of the present disclosure provides enhanced stability, antioxidant benefits, and antimicrobial properties for application on skin, hair, nails, and/or scalp.

In an embodiment for better results, the cosmetic composition may comprise:
0.01 to 30% (w/w) of a bioactive mixture, preferably 10 to 30% (w/w), more preferably 15 to 20% (w/w);
0.01 to 50% (w/w) of a phospholipid as an emulsifier, preferably 20 to 50% (w/w), more preferably 25 to 40% (w/w);
0.01 to 40% (w/w) of a di-alcohol as a solvent, preferably 10 to 40% (w/w), more preferably 20 to 30% (w/w).

In an embodiment for better results, the phospholipid is selected from a list consisting of: phosphatidylcholine, phosphatidylethanolamine, phosphatidylinositol, phosphatidylserine, phosphatidic acid, lecithin including unsaturated as well as hydrogenated phospholipids, or mixtures thereof; preferably phosphatidylcholine, phosphatidylethanolamine, phosphatidylinositol, lecithin or mixtures thereof; lecithin more preferably phosphatidylcholine and /or lecithin.

In an embodiment for better results, the amount of an active cosmetic compound; ranges from 0.01 to 10 % (w/w), preferably 0.01 to 5 % (w/w), more preferably 0.5 to 2 % (w/w).

In an embodiment for better results, the at least two components of the bioactive mixture are selected from a list consisting of : lactic acid, glycolic acid, decanoic acid, citric acid, maleic acid, malic acid, oxalic acid, tartaric acid, oleic acid, palmitic acid, enanthic acid, formic acid, caprylic acid, ethylene glycol, propylene glycol, glycine, glycerol, glucose, transcutol, menthol, sodium lactate, sodium acetate, sodium citrate, xylitol and sorbitol more preferably lactic acid, glycolic acid, glycine, glycerol, ethylene glycol and propylene glycol, choline chloride, or mixtures thereof.

In an embodiment for better results, the di-alcohol is selected from a list consisting of : 1,2-Propanediol; 1,3-Propanediol; 1,2-Butanediol; 1,3-Butanediol; 1,4-Butanediol; 2,3-Butanediol; 1,2-Hexanediol; 1,6-Hexanodiol; 1,5-Pentanediol; 1,2-Octanediol; 1,8-Octanediol; 1,2-Decanediol; 1,10-Decanediol; Methylpropanediol; 2-Butyl-2-ethyl-1,3-propanediol, Isopentyldiol and Ethane-1,2-diol or mixtures thereof, preferably 1,2-Propanediol; 1,3-Propanediol; 1,2-Butanediol; 1,3-Butanediol; 1,4-Butanediol; 2,3-Butanediol; 1,2-Hexanediol; 1,6-Hexanodiol; more preferably 1,3-Propanediol, 1,2-Butanediol and 1,2-Hexanediol, or mixtures thereof.

In an embodiment for better results, the composition may further comprise a natural oil; preferably an essential oil, an insect-based oil, a plant-based oil, or mixtures thereof.

In an embodiment for better results, the composition may further comprise a peptide preferably at least a peptide 80%, 85%, 90%, 91%, 92%, 93%, 94%, 95%, 96%, 97%, 98%, 99%, or 100% sequence identity to a sequence selected from a list consisting of:
SEQ ID No 552:YGGFL;
SEQ ID No 553: YPWF;
SEQ ID No 554: YGGFM;
SEQ ID No 555: YPFF;
SEQ ID No 556: EEMQRR;
SEQ ID No 557: MGWCTASVPPQCYG;
SEQ ID No 558: ATAKAWLKGIGAGAGAGA;
SEQ ID No 559: RRQMEEGAGAGAGA;
SEQ ID No 560: GGVCGPSPPCITTATAKAWLKGI;
SEQ ID No 561: GGVCGPSPPCITTGAGAGAGATAKAWLKGI;
SEQ ID No 562: ATAKAWLKGIGGVCGPSPPCITT;
SEQ ID No 563: ATAKAWLKGIAGAGAGAGGVCGPSPPCITT;
SEQ ID No 564: GGVCGPSPPCITTATAKAWLKGIRRQMEE;
SEQ ID No 565: RRQMEEATAKAWLKGIGGVCGPSPPCITT;
SEQ ID No 566: GGVCGPSPPCITTRRQMEE;
SEQ ID No 584: GGVCGPSPPCITTGAGAGAGARRQMEE;
SEQ ID No 585: RRQMEEGGVCGPSPPCITT;
SEQ ID No 586: RRQMEEGGVCGPSPPCITTGRKKRRQRRRPPQ;
SEQ ID No 587: GRKKRRQRRRPPQGGVCGPSPPCITTRRQMEE;SEQ ID No 568: RRQMEEGAGAGAGAGGVCGPSPPCITT;
SEQ ID No 569: ATAKAWLKGIMGWCTASVPPQCYG;
SEQ ID No 570: RRQMEEMGWCTASVPPQCYG;
SEQ ID No 2: GGVCGPSPPCITTVPGVGVPGVGVPGVGVPGVGVPGVGVPGVG;
SEQ ID No 549: GGVCGPSPPCITTGAGAGSGAGAGSVPAVGVPAVGVPAVG;
SEQ ID No 5: GGVCGPSPPCITTGPTGPTGPAGPRGLQGLQGLQGERGEQGPT;
SEQ ID No 4: GGVCGPSPPCITTGGRPSDSYGAPGGGN;
SEQ ID No 6: GGVCGPSPPCITTGGFGGMGGGSGGFGGMGGGSGGFGGMGGGS;
SEQ ID No 45: GGVCGPSPPCITTAKAKAKAKAKAKAKAKAKAKAKAKAKAKAK;
SEQ ID No 46: GGVCGPSPPCITTLKLKLKLKLKLKLKLKLKLKLKLKLKLKLK;
SEQ ID No 573: GGVCGPSPPCITTRCAERCSEASIQDRCLKYCGICCEK;
SEQ ID No 574: GGVCGPSPPCITTGTYGNKDECPCYRDMKNSKGKGKCP;
SEQ ID No 572: GGVCGPSPPCITTGTCYINDDEGCTKRCQGIYIYRREAVMGHC;
SEQ ID No 588: LKLKLKLKLKLKLKLKLKLK;
SEQ ID No 589: LELELELELELELELELELE;
SEQ ID No 571: YGRKKRRQRRR;
SEQ ID No 575: GRKKRRQRRRPPQ;
SEQ ID No 576: RQIKIWFQNRRMKWKK;
SEQ ID No 590: RKKNPNCRRH;
SEQ ID No 577: RIMRILRILKLAR;
SEQ ID No 578: LLIILRRRRIRKQAHAHSK;
SEQ ID No 579: GVCGPSPPCITTDRDDQAAWFSQY;
SEQ ID No 580: 
MVYYPNPCSPYPCRNGGTCKKRGLYSYKCYCRKGYTGKNCQYNACFPNPCLNGGTCGYVYG
YPYYKCSCPYGYYGKQCQLKKYGGVCGPSPPCITT;
SEQ ID No 581: GGVCG PSPPCITTERSRSSDG KSSSQVN RSRH E NTSQVPLQESRTRKRRGSRVSQD RDSEG H;
SEQ ID No 582: GGVCG PSPPCITTDGTRHSGSR H H EASSQADSSRH SQVGQGQSSG PRTSRNQGSSVSQDSD
SQGH; SEQ ID No 583: 
ERSRSSDGKSSSQVNRSRHENTSQVPLQESRTRKRRGSRVSQDRDSEGHGGVCGPSPPCITT
DGTRHSGSRHHEASSQADSSRHSQVGQGQSSGPRTSRNQGSSVSQDSDSQGH;
 or sequences or table 2 or 3 or mixtures thereof.

In an embodiment for better results, the amount peptide ranges from 0.0001 % to 20 % (w/w); preferably the amount peptide ranges from 0.001% to 5 % (w/w); or 0.1% to about 1% (w/w).

In an embodiment for better results, the composition may further comprise at least one excipient suitable for dermatological use.

In an embodiment for better results, the at least one excipient suitable for dermatological use is selected from the following list: preservative, thickener, organic polymer, humectant, silicone, oil, fragrance, vitamin, buffer, antimicrobial agent, antibacterial agent, disinfectant, chelating agent or mixtures thereof.

In an embodiment for better results, the at least one excipient suitable for dermatological use is selected from the following list: water, ethanol, benzyl alcohol, urea, ammonium thioglycolate, thioanisole, tris(hydroxymethyl)aminomethane, phosphate buffer, sodium hydroxide, sodium chloride, citrate buffer, or a combination of two or more thereof.

In an embodiment for better results, the composition may further comprise a propellant, a fragrance, an oil, or mixture thereof.

In an embodiment for better results, the composition of the present disclosure may be used in hair or scalp treatment.

In an embodiment for better results, the composition of the present disclosure may be used as a cosmetic product; namely as a cosmetic for hair, nails, skin or scalp.

In an embodiment for better results, the composition of the present disclosure may be a shampoo, a lotion, a serum, a cream, a conditioner, a foam, an elixir, an oil, an aerosol or a mask comprising the cosmetic composition described in the present disclosure. In an embodiment, the components of the described cosmetic composition may be enriched with active components for sensations of warm, cold, freshness, relaxing, pain relive, lightness and well-being, preferably selected from a list comprising: a peptide, icilin, menthol, carboxylated icilin, carboxylated menthol, baicalin, curcumin, caffeine, or mixtures thereof, among others.

Another aspect of the present disclosure relates to the use of a cosmetic composition as described in the present disclosure as a cosmetic product, namely as a cosmetic for hair, nails, skin or scalp.

Further particular and preferred aspects are set out in the accompanying independent and dependent claims. Features of the dependent claims may be combined with features of the independent claims as appropriate, and in combinations other than those explicitly set out in the claims.

### BRIEF DESCRIPTION OF THE DRAWINGS

The following figures provide preferred embodiments for illustrating the disclosure and should not be seen as limiting the scope of invention.

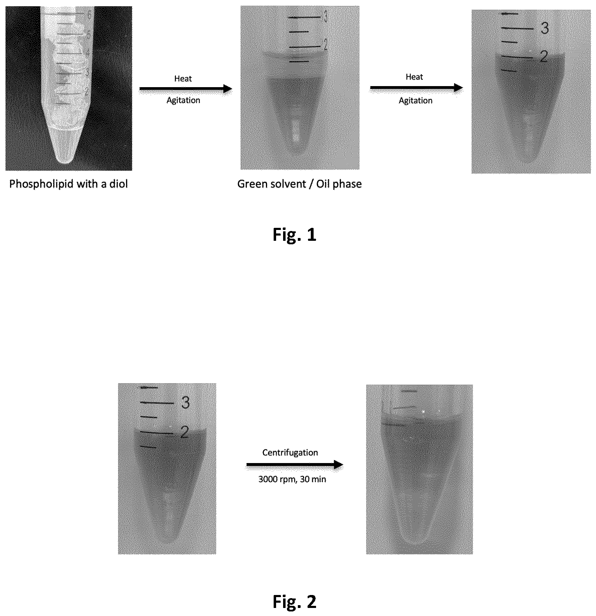

Figure 1: Schematic representation of a stable cosmetic formulation comprising a green solvent, a phospholipid as an emulsifier, an oil phase, and at least one soluble auxiliary, such as a diol.
Figure 2: Assessment of the stability of a cosmetic emulsified formulation by centrifugation (3000 rpm, 30 minutes at room temperature).

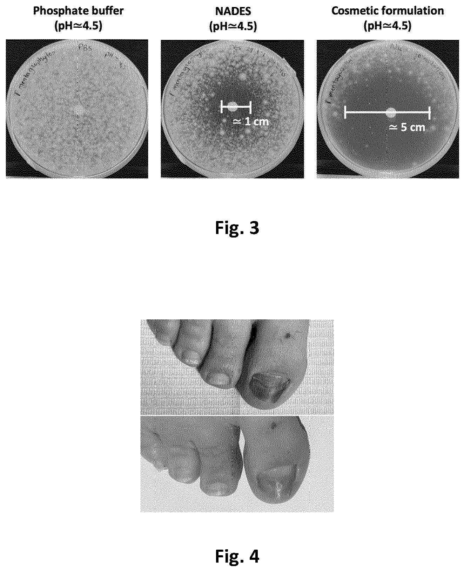

Figure 3: Anti-fungal activity of a cosmetic formulation against *Trichophyton mentagrophytes* strain.
Figure 4: Visual inspection of the anti-fungal activity of a cosmetic formulation in toenails.

### DETAILED DESCRIPTION

The present disclosure provides a stable emulsified cosmetic composition comprising a green solvent, an emulsifier, an oil phase, and at least one soluble auxiliary; preferably a waterless stable compositin. The green solvent can be a naturally occurring product, a mixture of natural solvents, or a Natural Deep Eutectic Solvent (NADES), serving either as a solvent or as a medium to extract bioactive compounds. The emulsifier, such as phospholipids, ensures the stability of the emulsion, while the oil phase delivers essential moisturizing properties. The composition may further include a peptide, a cosmetic component, or a combination thereof to target and enhance specific attributes of hair, nails, scalp, and skin. This composition provide long-lasting stability, antioxidant benefits, and antimicrobial properties, making it suitable for various cosmetic applications.

A stable emulsified cosmetic composition with enhanced stability, antioxidant benefits, and antimicrobial properties for application on skin, hair, nails, and/or scalp. The composition comprises a carefully selected blend of emulsifiers (e.g., phospholipids), antioxidants, and antimicrobial agents, ensuring the stability of the emulsion over time and promoting skin, hair, nails, and/or scalp health.

A stable emulsified cosmetic composition comprising a bioactive mixture, a phospholipid as an emulsifier, an oil component and at least one diol with enhanced stability, moisturizing effect, antioxidant benefits, and antimicrobial properties for application on skin, hair, nails, and/or scalp. The composition comprises a carefully selected blend of emulsifiers, antioxidants, and antimicrobial agents, ensuring the stability of the emulsion over time and promoting a suitable treatment for beauty healthcare.

### Example 1:

In example 1, it was prepared an emulsified cosmetic composition with:
10% (w/w) of rosemary NADES extract as a bioactive mixture (where NADES is glycerol:lactic acid, 4:1 molar ratio);
50% (w/w) of phosphatidylcholine as an emulsifier;
20% (w/w) of 1,3-Propanediol as a solvent;
20% (w/w) of tea tree oil as a bioactive oil phase.

### Example 2:

In example 2, it was prepared an emulsion with:
20% (w/w) of a thymol and carvacrol solution in NADES as a bioactive mixture (where NADES is glycerol:lactic acid, 4:1 molar ratio);
25% (w/w) of phosphatidylcholine as an emulsifier;
10% (w/w) of 1,2-Hexanediol as a solvent;
20% (w/w) of 1,3-Propanediol as a solvent;
25% (w/w) of tea tree oil as a bioactive oil phase;
0.01% (w/w) SEQ ID No 46: GGVCGPSPPCITT.

### Example 3:

In example 3, it was prepared an emulsion with:
10% (w/w) of lavender NADES extract as a bioactive mixture (where NADES is glycerol:lactic acid, 4:1 molar ratio);
50% (w/w) of lecithin as an emulsifier;
10% (w/w) of 1,2-Butanediol as a solvent;
10% (w/w) of 1,2-Propanediol as a solvent;
20% (w/w) of tea tree oil as a bioactive oil phase.

**Table 1: Assessment of the antioxidant activity by DPPH (2,2-Diphenyl-1-picrylhydrazyl) assay and quantification of total phenolic and flavonoid contents.**

| **Extraction method** | **Extraction Solvent** | **AA %** | **Total phenolic content** | **Total flavonoid content** | **Extraction Yield** |
|---|---|---|---|---|---|
| | | **(antioxidant activity %)** | **(mg of gallic acid/ g of extract)** | **(mg of quercetin/ g of extract)** | **(%)** |
| **Ultra-Turrex** | Insect-based extract with green solvent | 85.3 | 1.41 | 0.24 | 39.8 |
| | Green solvent | 76.7 | - | - | - |

**Table 2. Non-Limiting Example Peptide Sequences.**

| SEQ ID NO: | Sequences |
|---|---|
| Keratin/Keratin-like peptides (KLPs) | |
| 46 | GGVCGPSPPCITT |
| 9 | GGVCGPSPPC |
| 10 | CGPSPPCITT |
| 48 | |
| 66 | CGPSPPC |
| 67 | GVCGPSPPC |
| 68 | CLPCLPAASC |
| 69 | CLPAASC |
| 101 | VSSCCKPQCC |
| 102 | CCKPQCC |
| 103 | CCQSSCCKPS |
| 70 | YGGSSGGG |
| 71 | FGGGS |
| 72 | CCQSSCCKPSC |
| 73 | CVSSCCKPQCC |
| 74 | PITCRRTCYH |
| 75 | DCKLPCNPCA |
| 76 | CLPCLPAASC |
| 77 | CEPAICEPSC |
| 78 | CQCSCCKPYCS |
| 79 | FCGFPSCSTSC |
| 80 | CTPPSCCQLHHA |
| 81 | SCCAPVYCCK |

| Elastin/Elastin-like peptides (ELPs) | |
|---|---|
| 11 | VPGG |
| 12 | IPGG |
| 13 | VPAVG |
| 14 | AVGVP |
| 15 | IPGVG |
| 16 | LPGVG |
| 17 | VAPGVG |
| 18 | GVGVPGVG |
| 19 | VPGFGVGAG |
| 20 | VPGVGVPGG |
| 21 | VPGX²G |
| 22 | GVGVPGVGVPGLGVPGVGVPGVG |
| 23 | VPGVGVPGVGVPGVGVPGVGVPGVGVPGVG |
| 49 | (VPGXG)n, where n is 1 to 10 and each X is independently any amino acid |
| 50 | VPGVG |
| 51 | VPGLG |
| 52 | VPGVGVPGL |
| 53 | VPGVGVPGVGVPGLGVPGVGVPGVGR |
| 54 | VPGVGVPGVGVPG LGVPGVGVPGVG |
| 55 | LLNLAGLVPGVGVAPGVGVAPGVGVAPGVGLAPGVGVAPGVGVAPGVGVAPGIGPGGVAAAAKSAAKVA AKAQLRAAAGLGAGIPGLGVGVGVPGLGVGAGVPGLGVGAGVPGFGAVPGALAAAKAAKYGAAVPGVLG GLGALGGVGIPGGVVGAGPAAAAAAAKAAAKAAQFGLVGAAGLGGLGVGGLGVPGVGGLGGIPPAAAAK AAKYGAAGLGGVLGGAGQFPLGGVAARPGFGLSPIFPGGACLGKACGRKRK |
| 82 | IPGLG |
| 83 | GYGVP |
| 84 | GGGVP |
| 85 | IPGVGIPGLG |
| 86 | IPGVGVPGLG |
| 87 | VPGVGVPGLG |
| 88 | VPGVGIPGVG |
| 89 | VPGVGIPGLG |
| 90 | GYGVPGGGVP |
| 91 | IPGVGAVGVP |
| 104 | MAGLTAAAPRPGVLLLLLSILHPSRPGGVPGAIPGGVPGGVFYPGAGLGALGGGALGPGGKPLKPVPGGLAG AGLGAGLGAFPAVTFPGALVPGGVADAAAAYKAAKAGAGLGGVPGVGGLGVSAGAVVPQPGAGVKPGKV PGVGLPGVYPGGVLPGARFPGVGVLPGVPTGAGVKPKAPGVGGAFAGIPGVGPFGGPQPGVPLGYPIKAPK LPGGYGLPYTTGKLPYGYGPGGVAGAAGKAGYPTGTGVGPQAAAAAAAKAAAKFGAGAAGVLPGVGGAG VPGVPGAIPGIGGIAGVGTPAAAAAAAAAKAAKYGAAAGLVPGGPGFGPGVVGVPGAGVPGVGVPGAGI PVVPGAGIPGAAVPGVVSPEAAAKAAAKAAKYGARPGVGVGGIPTYGVGAGGFPGFGVGVGGIPGVAGVP |
| | GVGGVPGVGGVPGVGISPEAQAAAAAKAAKYGAAGAGVLGGLVPGAPGAVPGVPGTGGVPGVGTPAAA AAKAAAKAAQFGLVPGVGVAPGVGVAPGVGVAPGVGLAPGVGVAPGVGVAPGVGVAPGIGPGGVAAAA KSAAKVAAKAQLRAAAGLGAGIPGLGVGVGVPGLGVGAGVPGLGVGAGVPGFGAGADEGVRRSLSPELRE GDPSSSQHLPSTPSSPRVPGALAAAKAAKYGAAVPGVLGGLGALGGVGIPGGVVGAGPAAAAAAAKAAAK AAQFGLVGAAGLGGLGVGGLGVPGVGGLGGIPPAAAAKAAKYGAAGLGGVLGGAGQFPLGGVAARPGFG LSPIFPGGACLGKACGRKRK |

| Silk/Silk-like peptides (SLPs) | |
|---|---|
| 24 | GAGAGS |
| 25 | GAGSGA |
| 26 | GAGAGY |
| 27 | GAGYGA |
| 28 | GAGAGA |
| 29 | GAGAGV |
| 30 | GAGVGA |
| 31 | GAGAGVGY |
| 32 | GAGAGSGAGAGSGAGAGSGAGAGSGAGAGS |
| 56 | GYGAGAGSGAASGAGAGSGAGAGSGAGAGSGAGAGSGAGAGSGAGAGSGAGAG SGAGAGYGAGAGVGYGAGAGSGAASGAGAGSGAGAGSGAGAGSGAGAGS |
| 105 | MRVKTFVILCCALQYVAYTNANINDFDEDYFGSDVTVQSSNTTDEIIRDASGAVIEEQITTKKMQRKNKNHGI LGKNEKMIKTFVITTDSDGNESIVEEDVLMKTLSDGTVAQSYVAADAGAYSQSGPYVSNSGYSTHQGYTSDF STSAAVGAGAGAGAAAGSGAGAGAGYGAASGAGAGAGAGAGYGTGAGAGAGAGYGAGAGAGAGA GYGAGAGAGAGAGYGAGAGAGAGAGYGAGAGAGAGAGYGAGAGAGAGAGYGAASGAGAGAGYGQG VGSGAASGAGAGAGAGSAAGSGAGAGAGTGAGAGYGAGAGAGAGAGYGAASGTGAGYGAGAGAGYG GASGAGAGAGAGAGAGAGYGTGAGYGAGAGAGAGAGAGYGAGAGAGYGAGYGVGAGAGYGA GYGAGAGSGAASGAGSGAGAGSGAGAGSGAGAGSGAGAGSGAGAGSGAGAGSGAGAGSGAGAGSGTG AGSGAGAGYGAGAGAGYGAGAGSGAASGAGAGSGAGAGSGAGAGSGAGAGSGAGAGSGAGAGYGAG AGAGYGAGAGAGYGAGAGVGYGAGAGSGAASGAGAGSGAGAGSGAGAGSGAGAGSGAGAGSGAGAG SGAGAGSGAGAGSGAGAGSGAGVGYGAGVGAGYGAGYGAGAGAGYGAGAGSGAASGAGAGAGAGAG TGSSGFGPYVANGGYSRSDGYEYAWSSDFGTGSGAGAGSGAGAGSGAGAGSGAGAGSGAGAGSGAGAG YGAGVGVGYGAGYGAGAGAGYGAGAGSGAASGAGAGSGAGAGSGAGAGSGAGAGSGAGAGSGAGAG SGAGAGSGAGAGSGAGAGSGAGAGSGAGVGSGAGAGSGAGAGVGYGAGAGVGYGAGAGSGAASGAG AGSGAGAGSGAGAGSGAGAGSGAGAGSGAGAGSGAGAGSGAGAGSGAGAGSGAGVGYGAGVGAGYG AGYGAGAGAGYGAGAGSGAASGAGAGSGAGAGSGAGAGSGAGAGSGAGAGSGAGAGSGAGAGSGAG AGSGAGAGSGAGAGSGAGAGSGAGAGYGAGAGAGYGAGYGAGAGAGYGAGAGSGAASGAGSGAGAG SGAGAGAGSGAGAGSGAGAGSGAGAGSGAGAGSGAGAGSGAGAGYGAGVGAGYGAGYGAGAGAGYG AGAGSGAASGAGAGSGAGAGSGAGAGSGAGAGSGAGAGSGAGAGSGAGAGSGAGVGYGAGYGAGAG AGYGAGAGSGAASGAGAGAGAGAGTGSSGFGPYVAHGGYSGYEYAWSSESDFGTGSGAGAGSGAGAGS GAGAGSGAGAGSGAGYGAGVGAGYGAGYGAGAGAGYGAGAGSGAGSGAGAGSGAGAGSGAGAGSGA GAGSGAGAGSGAGAGSGAGAGSGAGAGSGAGAGYGAGYGAGAGAGYGAGAGSGAGSGAGAGSGAGA GSGAGAGSGAGAGSGAGAGSGAGAGSGAGAGSGAGAGYGAGVGAGYGAGYGAGAGAGYGAGAGSGA GSGAGAGSGAGAGSGAGAGSGAGVGSGAGAGSGAGAGSGAGAGSGAGAGYGAGYGAGAGAGYGAGA GSGAGSGAGAGSGAGAGSGAGAGSGAGAGSGAGAGSGAGAGSGAGAGSGAGVGYGAGVGAGYGAGY GAGAGAGYGAGAGSGAASGAGAGAGAGAGTGSSGFGPYVANGGYSGYEYAWSSESDFGTGSGAGAGSG AGAGSGAGAGSGAGAGSGAGAGYGAGYGAGAGAGYGAGAGSGAGSGAGAGSGAGAGSGAGAGSGAG AGSGAGAGSGAGAGSGAGAGSGAGSGSGAGAGSGAGAGSGAGAGYGAGVGAGYGVGYGAGAGAGYG AGAGSGAASGAGAGAGAGAGTGSSGFGPYVAHGGYSGYEYAWSSESDFGTGSGAGAGSGAGAGSGAGA GSGAGAGSGAGAGSGAGAGSGAGAGYGAGVGAGYGAAYGAGAGAGYGAGAGSGAASGAGAGSGAGA GSGAGAGSGAGAGSGAGAGSGAGAGSGAGAGSGAGAGSGAGAGSGAGAGSGAGAGYGAGAGAGYGA GAGSGAGSGAGAGSGAGAGSGAGAGSGAGAGSGAGAGSGAGAGSGAGSGSGAGAGSGAGAGSGAGAGYGAGV GAGYGAGYGAGAGAGYGAGAGSGAGSGAGAGSGAGAGYGAGAGAGYGAGYGAGAGAGYGAGAGTGA GSGAGAGSGAGAGSGAGAGSGAGAGSGAGAGSGAGAGSGAGAGSGAGSGSGAGAGSGAGAGSGAGAGSGAGA GSGAGAGSGAGAGYGAGAGAGYGAGYGAGAGAGYGAGAGSGAGSGAGAGSGAGAGSGAGAGSGAGA GYGAGYGAGAGSGAASGAGAGAGAGAGTGSSGFGPYVAHGGYSGYEYAWSSESDFGTGSGAGAGSGAG AGAGAGAGSGAGAGYGAGVGAGYGAGYGAGAGAGYGAGAGSGTGSGAGAGSGAGAGYGAGVGAGYG AGAGSGAAFGAGAGAGAGSGAGAGSGAGAGSGAGAGSGAGAGSGAGAGYGAGYGAGVGAGYGAGAG |
| | SGAASGAGAGSGAGAGSGAGAGSGAGAGSGAGAGSGAGAGYGAGVGAGYGAGYGAGAGAGYGAGAG SGAASGAGAGSGAGAGAGSGAGAGSGAGAGSGAGAGSGAGSGAGAGSGAGAGSGAGAGYGAGAGSG AASGAGAGAGAGAGTGSSGFGPYVANGGYSGYEYAWSSESDFGTGSGAGAGSGAGAGSGAGAGSGAGA GSGAGAGYGAGVGAGYGAGYGAGAGAGYGAGAGSGAGSGAGAGSGAGAGSGAGAGSGAGAGSGAGA GSGAGAGSGAGAGYGAGAGSGAASGAGAGSGAGAGSGAGAGSGAGAGSGAGAGSGAGAGSGAGAGY GAGVGAGYGVGYGAGAGAGYGAGAGSGAGSGAGAGSGAGAGSGAGAGSGAGAGSGAGSGAGAGSGA GAGSGAGAGSGAGSGAGAGSGAGAGYGVGYGAGAGAGYGAGAGSGAGSGAGAGSGAGAGSGAGAGS GAGSGAGAGSGAGAGSGAGAGSGAGAGYGAGVGAGYGVGYGAGAGAGYGAGAGSGAGSGAGAGSGA GAGSGAGAGSGAGAGSGAGAGSGAGAGSGAGAGSGAGSGAGAGSGAGAGSGAGAGSGAGAGSGAGS GAGAGSGAGAGSGAGAGSGAGAGYGAGVGAGYGVGYGAGVGAGYGAGAGSGAASGAGAGSGAGAGA GSGAGAGSGAGAGSGAGAGSGAGAGSGAGAGSGAGAGYGAGYGAGVGAGYGAGAGVGYGAGAGAGY GAGAGSGAASGAGAGAGSGAGAGTGAGAGSGAGAGYGAGAGSGAASGAGAGAGAGAGTGSSGFGPYV ANGGYSGYEYAWSSESDFGTGSGAGAGSGAGAGSGAGAGSGAGAGSGAGAGYGAGVGAGYGAGAGSG AGSGAGAGSGAGAGSGAGAGSGAGAGSGAGAGYGAGAGSGTGSGAGAGSGAGAGSGAGAGSGAGAGS GAGAGSGAGAGSGVGAGYGVGYGAGAGAGYGVGYGAGAGAGYGAGAGSGTGSGAGAGSGAGAGSGA GAGSGAGAGSGAGAGSGAGAGSGAGAGYGAGVGAGYGVGYGAGAGAGYGAGAGSGAGSGAGAGSGA GAGSGAGAGSGAGAGSGAGSGAGAGSGAGAGSGAGAGSGAGSGAGAGSGAGAGYGVGYGAGAGAGY GAGAGSGAGSGAGAGSGAGAGSGAGAGSGAGSGAGAGSGAGAGSGAGAGSGAGAGYGAGVGAGYGV GYGAGAGAGYGAGAGSGAGSGAGAGSGAGAGSGAGAGSGAGAGSGAGAGSGAGAGSGAGAGSGAGS GAGAGSGAGAGSGAGAGSGAGAGYGAGVGAGYGVGYGAGAGAGYGAGAGSGAASGAGAGAGAGAGT GSSGFGPYVANGGYSGYEYAWSSESDFGTGSGAGAGSGAGAGSGAGAGYGAGYGAGVGAGYGAGAGVG YGAGAGAGYGAGAGSGAASGAGAGAGAGSGAGAGSGAGAGAGSGAGAGYGAGYGIGVGAGYGAG AGVGYGAGAGAGYGAGAGSGAASGAGAGSGAGAGSGAGAGSGAGAGSGAGAGSGAGAGSGAGAGYG AGYGAGVGAGYGAGAGVGYGAGAGAGYGAGAGSGAASGAGAGAGAGAGAGSGAGAGSGAGAGSGAG AGSGAGAGSGAGAGSGAGAGSGAGAGSGAGAGSGAGAGYGAGVGAGYGAGYGGAGAGYGAGAGSGA ASGAGAGSGAGAGSGAGAGSGAGAGSGAGAGSGAGAGYGAGAGSGAASGAGAGAGAGAGTGSSGFGP YVNGGYSGYEYAWSSESDFGTGSGAGAGSGAGAGSGAGAGYGAGVGAGYGAGYGAGAGAGYGAGAGS GAASGAGAGSGAGAGSGAGAGSGAGAGSGAGSGAGAGSGAGAGSGAGAGSGAGAGSGAGAGSGAGA GYGAGVGAGYGAGYGAGAGAGYGAGAGSGAASGAGAGSGAGAGAGSGAGAGSGAGAGSGAGAGSGA GAGSGAGAGSGAGSGAGAGSGAGAGYGAGYGAGVGAGYGAGAGVGYGAGAGAGYGAGAGSGAASGA GAGSGSGAGSGAGAGSGAGAGSGAGAGAGSGAGAGSGAGAGSGAGAGYGAGYGAGAGSGAASGAGA GAGAGAGTGSSGFGPYVANGGYSGYEYAWSSESDFGTGSGAGAGSGAGAGSGAGAGYGAGVGAGYGAG YGAGAGAGYGAGAGSGAGSGAGAGSGAGAGSGAGAGSGAGAGSGAGAGSGAGAGSGAGAGSGAGAG YGAGYGAGAGAGYGAGAGVGYGAGAGAGYGAGAGSGAGSGAGAGSGSGAGAGSGSGAGSGAGAGSG AGAGSGAGAGSGAGAGSGAGAGSGAGAGSGAGAGYGAGYGIGVGAGYGAGAGVGYGAGAGAGYGAG AGSGAASGAGAGSGAGAGSGAGAGSGAGAGSGAGAGSGAGAGSGAGAGSGAGAGSGAGAGSGAGAG YGAGAGVGYGAGAGSGAASGAGAGSGAGAGSGAGAGSGAGAGSGAGAGSGAGAGSGAGAGSGAGSG AGAGSGAGAGYGAGYGAGVGAGYGAGAGYGAGYGVGAGAGYGAGAGSGAGSGAGAGSGAGAGSGAG AGSGAGAGSGAGAGSGAGSGAGAGYGAGAGAGYGAGAGAGYGAGAGSGAASGAGAGAGSGAGAG SGAGAGSGAGSGAGAGSGAGAGYGAGAGSGAASGAGAGSGAGAGAGAGAGSGAGAGSGAGAGYG AGAGSGAASGAGAGAGAGTGSSGFGPYVANGGYSRREGYEYAWSSKSDFETGSGAASGAGAGAGSGAGA GSGAGAGSGAGAGSGAGAGGSVSYGAGRGYGQGAGSAASSVSSASSRSYDYSRRNVRKNCGIPRRQLVVK FRALPCVNC |
| 92 | GAGX¹GX², wherein X¹ and X² are independently any amino acid, optionally wherein X¹ is A, S, Y, or V, optionally wherein X² is S, A, Y, or V |

| Collagen/Collagen-like peptides (CLPs) | |
|---|---|
| 33 | GPTGPTGPAGPRGLQGLQGLQGERGEQGPT |
| 57 | GPTGPT |
| 58 | GLQGLQ |
| 59 | MFSFVDLRLLLLLAATALLTHGQEEGQVEGQDEDIPPITCVQNGLRYHDRDVWKPEPCRICVCDNGKVLCDD VICDETKNCPGAEVPEGECCPVCPDGSESPTDQETTGVEGPKGDTGPRGPRGPAGPPGRDGIPGQPGLPGP PGPPGPPGPPGLGGNFAPQLSYGYDEKSTGGISVPGPMGPSGPRGLPGPPGAPGPQGFQGPPGEPGEPGA SGPMGPRGPPGPPGKNGDDGEAGKPGRPGERGPPGPQGARGLPGTAGLPGMKGHRGFSGLDGAKGDA GPAGPKGEPGSPGENGAPGQMGPRGLPGERGRPGAPGPAGARGNDGATGAAGPPGPTGPAGPPGFPGA VGAKGEAGPQGPRGSEGPQGVRGEPGPPGPAGAAGPAGNPGADGQPGAKGANGAPGIAGAPGFPGAR |
| | GPSGPQGPGGPPGPKGNSGEPGAPGSKGDTGAKGEPGPVGVQGPPGPAGEEGKRGARGEPGPTGLPGPP GERGGPGSRGFPGADGVAGPKGPAGERGSPGPAGPKGSPGEAGRPGEAGLPGAKGLTGSPGSPGPDGKT GPPGPAGQDGRPGPPGPPGARGQAGVMGFPGPKGAAGEPGKAGERGVPGPPGAVGPAGKDGEAGAQG PPGPAGPAGERGEQGPAGSPGFQGLPGPAGPPGEAGKPGEQGVPGDLGAPGPSGARGERGFPGERGVQ GPPGPAGPRGANGAPGNDGAKGDAGAPGAPGSQGAPGLQGMPGERGAAGLPGPKGDRGDAGPKGAD GSPGKDGVRGLTGPIGPPGPAGAPGDKGESGPSGPAGPTGARGAPGDRGEPGPPGPAGFAGPPGADGQP GAKGEPGDAGAKGDAGPPGPAGPAGPPGPIGNVGAPGAKGARGSAGPPGATGFPGAAGRVGPPGPSGN AGPPGPPGPAGKEGGKGPRGETGPAGRPGEVGPPGPPGPAGEKGSPGADGPAGAPGTPGPQGIAGQRGV VGLPGQRGERGFPGLPGPSGEPGKQGPSGASGERGPPGPMGPPGLAGPPGESGREGAPGAEGSPGRDGS PGAKGDRGETGPAGPPGAPGAPGAPGPVGPAGKSGDRGET |
| 93 | GXPGXP (each X is independently any amino acid) |
| 94 | GZPGZP (each Z is 4-hydroxyproline) |
| 95 | GPAGPA |
| 106 | MLGTLTPLSSLLLLLLVLVLGCGPRASSGGGAGGAAGYAPVKYIQPMQKGPVGPPFREGKGQYLEMPLPLLP MDLKGEPGPPGKPGPRGPPGPPGFPGKPGMGKPGLHGQPGPAGPPGFSRMGKAGPPGLPGKVGPPGQP GLRGEPGIRGDQGLRGPPGPPGLPGPSGITIPGKPGAQGVPGPPGFQGEPGPQGEPGPPGDRGLKGDNGV GQPGLPGAPGQGGAPGPPGLPGPAGLGKPGLDGLPGAPGDKGESGPPGVPGPRGEPGAVGPKGPPGVD GVGVPGAAGLPGPQGPSGAKGEPGTRGPPGLIGPTGYGMPGLPGPKGDRGPAGVPGLLGDRGEPGEDGE PGEQGPQGLGGPPGLPGSAGLPGRRGPPGPKGEAGPGGPPGVPGIRGDQGPSGLAGKPGVPGERGLPGA HGPPGPTGPKGEPGFTGRPGGPGVAGALGQKGDLGLPGQPGLRGPSGIPGLQGPAGPIGPQGLPGLKGEP GLPGPPGEGRAGEPGTAGPTGPPGVPGSPGITGPPGPPGPPGPPGAPGAFDETGIAGLHLPNGGVEGAVLG KGGKPQFGLGELSAHATPAFTAVLTSPFPASGMPVKFDRTLYNGHSGYNPATGIFTCPVGGVYYFAYHVHVK GTNVWVALYKNNVPATYTYDEYKKGYLDQASGGAVLQLRPNDQVWVQMPSDQANGLYSTEYIHSSFSGFL LCPT |

| Resilin/Resilin-like peptides (RLP) | |
|---|---|
| 34 | GGRPSDSYGAPGGGN |
| 35 | GAPAQTPSSQY |
| 60 | MFKLLGLTLLMAMVVLGRPEPPVNSYLPPSDSYGAPGQSGPGGRPSDSYGAPGGGNGGRPSDSYGAPGQG QGQGQGQGGYAGKPSDTYGAPGGGNGNGGRPSSSYGAPGGGNGGRPSDTYGAPGGGNGGRPSDTYGA PGGGGNGNGGRPSSSYGAPGQGQGNGNGGRSSSSYGAPGGGNGGRPSDTYGAPGGGNGGRPSDTYGA PGGGNNGGRPSSSYGAPGGGNGGRPSDTYGAPGGGNGNGSGGRPSSSYGAPGQGQGGFGGRPSDSYGA PGQNQKPSDSYGAPGSGNGNGGRPSSSYGAPGSGPGGRPSDSYGPPASGSGAGGAGGSGPGGADYDND EPAKYEFNYQVEDAPSGLSFGHSEMRDGDFTTGQYNVLLPDGRKQIVEYEADQQGYRPQIRYEGDANDGS GPSGPGGPGGQNLGADGYSSGRPGNGNGNGNGGYSGGRPGGQDLGPSGYSGGRPGGQDLGAGGYSNG KPGGQDLGPGGYSGGRPGGQDLGRDGYSGGRPGGQDLGASGYSNGRPGGNGNGGSDGGRVIIGGRVIG GQDGGDQGYSGGRPGGQDLGRDGYSSGRPGGRPGGNGQDSQDGQGYSSGRPGQGGRNGFGPGGQNG DNDGSGYRY |
| 96 | AQTPSSQYGAP |
| 107 | MFKLLGLTLLMAMVVLGRPEPPVNSYLPPSDSYGAPGQSGPGGRPSDSYGAPGGGNGGRPSDSYGAPGQG QGQGQGQGGYAGKPSDTYGAPGGGNGNGGRPSSSYGAPGGGNGGRPSDTYGAPGGGNGGRPSDTYGA PGGGGNGNGGRPSSSYGAPGQGQGNGNGGRSSSSYGAPGGGNGGRPSDTYGAPGGGNGGRPSDTYGA PGGGNNGGRPSSSYGAPGGGNGGRPSDTYGAPGGGNGNGSGGRPSSSYGAPGQGQGGFGGRPSDSYGA PGQNQKPSDSYGAPGSGNGNGGRPSSSYGAPGSGPGGRPSDSYGPPASGSGAGGAGGSGPGGADYDND EPAKYEFNYQVEDAPSGLSFGHSEMRDGDFTTGQYNVLLPDGRKQIVEYEADQQGYRPQIRYEGDANDGS GPSGPGGPGGQNLGADGYSSGRPGNGNGNGNGGYSGGRPGGQDLGPSGYSGGRPGGQDLGAGGYSNG KPGGQDLGPGGYSGGRPGGQDLGRDGYSGGRPGGQDLGASGYSNGRPGGNGNGGSDGGRVIIGGRVIG GQDGGDQGYSGGRPGGQDLGRDGYSSGRPGGRPGGNGQDSQDGQGYSSGRPGQGGRNGFGPGGQNG DNDGSGYRY |

| Abductin/Abductin-like peptides (ALP) | |
|---|---|
| 36 | GGFGGMGGGS |
| 37 | MGGG |
| 38 | FGGMG |
| 39 | FGGMGGG |
| 40 | GGFGGMGGG |
| 41 | FGGMGGGNAG |
| 42 | GGFGGMGGGSGGFGGMGGGSGGFGGMGGGS |
| 61 | MNAYICLAACLIAAVSAAGYGGGAGSMGGTGGMGGGMNAGGFGGMGGMGGGKGGFGGIGGFGGGM GGGPGGFGGMGGFGGMAAKGGFGGMGSGMGGFGGMGGGNAGFGGMGGGNAGFGGMGGQGGFG GKGY |
| 108 | GGRPSDSYGAPGGGNGGRPSDSYGAPGGGNGGRPSDSYGAPGGGNGGRPSDSYGAPGGGNGGRPSDSY GAPGGGNGGRPSDSYGAPGGGNGGRPSDSYGAPGGGNGGRPSDSYGAPGGGNGGRPSDSYGAPGGGN GGRPSDSYGAPGGGNGQGQGQGQGQGQGQGQGQGGVCGPSPPCITTGQGQGQGQGQGQGQGQ GQGQVPAVGVPAVGVPAVGVPAVGVPAVGVPAVGVPAVGVPAVGVPAVGVPAVGGQGQGQGQGQG QGQGQGQGQGGVCGPSPPCITTGQGQGQGQGQGQGQGQGQGGRPSDSYGAPGGGNGGRPSDSY GAPGGGNGGRPSDSYGAPGGGNGGRPSDSYGAPGGGNGGRPSDSYGAPGGGNGGRPSDSYGAPGGGN GGRPSDSYGAPGGGNGGRPSDSYGAPGGGNGGRPSDSYGAPGGGNGGRPSDSYGAPGGGN |

| Linker/Spacer-like peptides | |
|---|---|
| 97 | GQGQGQGQGQGQGQGQGQGQGQGQGQGQGQ |
| 98 | AKAKAKAKAKAKAKAKAKAKAKAKAKAKAK |
| 99 | LKLKLKLKLKLKLKLKLKLKLKLKLKLKLK |
| 100 | GAGAGAGAGAGAGAGAGAGAGAGAGAGAGA |
| | (GQ)n, where n is 1-20 |
| | (AK)n, where n is 1-20 |
| | (LK)n, where n is 1-20 |
| | (GA)n, where n is 1-20 |

**Table 3. Non-Limiting Example Peptide sequences that can be added to the composition of the present disclosure.**

| SEQ ID NO: | Sequences |
|---|---|
| Elastin/ELP and Keratin Fusion Proteins | |
| 1 | GGVCGPSPPCITTVPGVGVPGVGVPGLGVPGVGVPGVGR |
| 2 | GGVCGPSPPCITTVPGVGVPGVGVPGVGVPGVGVPGVGVPGVG |
| 7 | GGVCGPSPPCITTVPGVGVPGVGVPGLGVPGVGVPGVG |
| 200 | GGVCGPSPPCVPGVGVPGVGVPGLGVPGVGVPGVGR |
| 201 | GGVCGPSPPCVPGVGVPGVGVPGVGVPGVGVPGVGVPGVG |
| 202 | GGVCGPSPPCVPGVGVPGVGVPGLGVPGVGVPGVG |
| 203 | CGPSPPCITTVPGVGVPGVGVPGLGVPGVGVPGVGR |
| 204 | CG PSP PCITTVPGVGVPGVGVPGVGVPGVGVPGVGVPGVG |
| 205 | CGPSPPCITTVPGVGVPGVGVPGLGVPGVGVPGVG |
| 206 | YGGSSGGGVPGVGVPGVGVPGLGVPGVGVPGVGR |
| 207 | YGGSSGGGVPGVGVPGVGVPGVGVPGVGVPGVGVPGVG |
| 208 | YGGSSGGGVPGVGVPGVGVPGLGVPGVGVPGVG |
| 209 | FGGGSVPGVGVPGVGVPGLGVPGVGVPGVGR |
| 210 | FGGGSVPGVGVPGVGVPGVGVPGVGVPGVGVPGVG |
| 211 | FGGGSVPGVGVPGVGVPGLGVPGVGVPGVG |
| 212 | CCQSSCCKPSCVPGVGVPGVGVPGLGVPGVGVPGVGR |
| 213 | CCQSSCCKPSCVPGVGVPGVGVPGVGVPGVGVPGVGVPGVG |
| 214 | CCQSSCCKPSCVPGVGVPGVGVPGLGVPGVGVPGVG |
| 215 | CVSSCCKPQCCVPGVGVPGVGVPGLGVPGVGVPGVGR |
| 216 | CVSSCCKPQCCVPGVGVPGVGVPGVGVPGVGVPGVGVPGVG |
| 217 | CVSSCCKPQCCVPGVGVPGVGVPGLGVPGVGVPGVG |
| 218 | P ITCR RTCYHVPGVGVPGVGVPG LGVPGVGVPGVG R |
| 219 | PITCRRTCYHVPGVGVPGVGVPGVGVPGVGVPGVGVPGVG |
| 220 | PITCR RTCYHVPGVGVPGVGVPGLGVPGVGVPGVG |
| 221 | DCKLPCNPCAVPGVGVPGVGVPGLGVPGVGVPGVGR |
| 222 | DCKLPCNPCAVPGVGVPGVGVPGVGVPGVGVPGVGVPGVG |
| 223 | DCKLPCNPCAVPGVGVPGVGVPGLGVPGVGVPGVG |
| 224 | CLPCLPAASCVPGVGVPGVGVPGLGVPGVGVPGVGR |
| 225 | CLPCLPAASCVPGVGVPGVGVPGVGVPGVGVPGVGVPGVG |
| 226 | CLPCLPAASCVPGVGVPGVGVPGLGVPGVGVPGVG |
| 227 | CEPAICEPSCVPGVGVPGVGVPGLGVPGVGVPGVGR |
| 228 | CEPAICEPSCVPGVGVPGVGVPGVGVPGVGVPGVGVPGVG |
| 229 | CEPAICEPSCVPGVGVPGVGVPGLGVPGVGVPGVG |
| 230 | CQCSCCKPYCSVPGVGVPGVGVPGLGVPGVGVPGVGR |
| 231 | CQCSCCKPYCSVPGVGVPGVGVPGVGVPGVGVPGVGVPGVG |
| 232 | CQCSCCKPYCSVPGVGVPGVGVPGLGVPGVGVPGVG |
| 233 | FCGFPSCSTSCVPGVGVPGVGVPGLGVPGVGVPGVGR |
| 234 | FCGFPSCSTSCVPGVGVPGVGVPGVGVPGVGVPGVGVPGVG |
| 235 | FCGFPSCSTSCVPGVGVPGVGVPGLGVPGVGVPGVG |
| 236 | CTPPSCCQLHHAVPGVGVPGVGVPGLGVPGVGVPGVGR |
| 237 | CTPPSCCQLHHAVPGVGVPGVGVPGVGVPGVGVPGVGVPGVG |
| 238 | CTPPSCCQLHHAVPGVGVPGVGVPGLGVPGVGVPGVG |
| 239 | SCCAPVYCCKVPGVGVPGVGVPGLGVPGVGVPGVGR |
| 240 | SCCAPVYCCKVPGVGVPGVGVPGVGVPGVGVPGVGVPGVG |
| 241 | SCCAPVYCCKVPGVGVPGVGVPGLGVPGVGVPGVG |
| 242 | GGVCGPSPPCITT(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 243 | GGVCGPSPPC(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 244 | CGPSPPCITT(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 245 | YGGSSGGG(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 246 | FGGGS(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 247 | CCQSSCCKPSC(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 248 | CVSSCCKPQCC(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 249 | PITCRRTCYH(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 250 | DCKLPCNPCA(X), wherein Xcomprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 251 | CLPCLPAASC(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 252 | CEPAICEPSC(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 253 | CQCSCCKPYCS(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 254 | FCGFPSCSTSC(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 255 | CTPPSCCQLHHA(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 256 | SCCAPVYCCK(X), wherein X comprises VPGG, IPGG, VPAVG, AVGVP, IPGVG, LPGVG, VAPGVG, GVGVPGVG, VPGFGVGAG, VPGVGVPGG, GVGVPGVGVPGLGVPGVGVPGVG, VPGVG, VPGLG, VPGVGVPGL, IPGLG, GYGVP, GGGVP, IPGVGIPGLG, IPGVGVPGLG, VPGVGVPGLG, VPGVGIPGVG, VPGVGIPGLG, GYGVPGGGVP, or IPGVGAVGVP, or a combination of two or more thereof |
| 257 | GGVCGPSPPCITT(VPGXG)n, where n is 1 to 10 and each X is independently any amino acid, optionally, wherein each X is independently V or L, and further optionally n is about 5 |
| 258 | GGVCGPSPPC(VPGXG)n, where n is 1 to 10 and each X is independently any amino acid, optionally, wherein each X is independently V or L, and further optionally n is about 5 |
| 259 | CGPSPPCITT(VPGXG)n, where n is 1 to 10 and each X is independently any amino acid, optionally, wherein each X is independently V or L, and further optionally n is about 5 |
| 260 | YGGSSGGG(VPGXG)n, where n is 1 to 10 and each X is independently any amino acid, optionally, wherein each X is independently V or L, and further optionally n is about 5 |
| 261 | FGGGS(VPGXG)n, where n is 1 to 10 and each X is independently any amino acid, optionally, wherein each X is independently V or L, and further optionally n is about 5 |
| 262 | CCQSSCCKPSC(VPGXG)n, where n is 1 to 10 and each X is independently any amino acid, optionally, wherein each X is independently V or L, and further optionally n is about 5 |
| 263 | CVSSCCKPQCC(VPGXG)n, where n is 1 to 10 and each X is independently any amino acid, optionally, wherein each X is independently V or L, and further optionally n is about 5 |
| 264 | PITCRRTCYH(VPGXG)n, where n is 1 to 10 and each X is independently any amino acid, optionally, wherein each X is independently V or L, and further optionally n is about 5 |
| 265 | DCKLPCNPCA(VPGXG)n, where n is 1 to 10 and each X is independently any amino acid, optionally, wherein each X is independently V or L, and further optionally n is about 5 |
| 266 | CLPCLPAASC(VPGXG)n, where n is 1 to 10 and each X is independently any amino acid, optionally, wherein each X is independently V or L, and further optionally n is about 5 |
| 267 | CEPAICEPSC(VPGXG)n, where n is 1 to 10 and each X is independently any amino acid, optionally, wherein each X is independently V or L, and further optionally n is about 5 |
| 268 | CQCSCCKPYCS(VPGXG)n, where n is 1 to 10 and each X is independently any amino acid, optionally, wherein each X is independently V or L, and further optionally n is about 5 |
| 269 | FCGFPSCSTSC(VPGXG)n, where n is 1 to 10 and each X is independently any amino acid, optionally, wherein each X is independently V or L, and further optionally n is about 5 |
| 270 | CTPPSCCQLHHA(VPGXG)n, where n is 1 to 10 and each X is independently any amino acid, optionally, wherein each X is independently V or L, and further optionally n is about 5 |
| 271 | SCCAPVYCCK(VPGXG)n, where n is 1 to 10 and each X is independently any amino acid, optionally, wherein each X is independently V or L, and further optionally n is about 5 |

| Silk/SLP and Keratin Fusion Proteins | |
|---|---|
| 3 | GGVCGPSPPCITTGAGAGSGAGAGSGAGAGSGAGAGSGAGAGS |
| 272 | GGVCGPSPPCGAGAGSGAGAGSGAGAGSGAGAGSGAGAGS |
| 273 | CGPSPPCITTGAGAGSGAGAGSGAGAGSGAGAGSGAGAGS |
| 274 | YGGSSGGGGAGAGSGAGAGSGAGAGSGAGAGSGAGAGS |
| 275 | FGGGSGAGAGSGAGAGSGAGAGSGAGAGSGAGAGS |
| 276 | CCQSSCCKPSCGAGAGSGAGAGSGAGAGSGAGAGSGAGAGS |
| 277 | CVSSCCKPQCCGAGAGSGAGAGSGAGAGSGAGAGSGAGAGS |
| 278 | PITCRRTCYHGAGAGSGAGAGSGAGAGSGAGAGSGAGAGS |
| 279 | DCKLPCNPCAGAGAGSGAGAGSGAGAGSGAGAGSGAGAGS |
| 280 | CLPCLPAASCGAGAGSGAGAGSGAGAGSGAGAGSGAGAGS |
| 281 | CEPAICEPSCGAGAGSGAGAGSGAGAGSGAGAGSGAGAGS |
| 282 | CQCSCCKPYCSGAGAGSGAGAGSGAGAGSGAGAGSGAGAGS |
| 283 | FCGFPSCSTSCGAGAGSGAGAGSGAGAGSGAGAGSGAGAGS |
| 284 | CTPPSCCQLHHAGAGAGSGAGAGSGAGAGSGAGAGSGAGAGS |
| 285 | SCCAPVYCCKGAGAGSGAGAGSGAGAGSGAGAGSGAGAGS |
| 286 | GGVCGPSPPCITTGAGX1GX2, wherein X1 and X2 are independently any amino acid, optionally wherein X1 is A, S, Y, or V, optionally wherein X2 is S, A, Y, or V |
| 287 | GGVCGPSPPCGAGX1GX2, wherein X1 and X2 are independently any amino acid, optionally wherein X1 is A, S, Y, or V, optionally wherein X2 is S, A, Y, or V |
| 288 | CGPSPPCITTGAGX1GX2, wherein X1 and X2 are independently any amino acid, optionally wherein X1 is A, S, Y, or V, optionally wherein X2 is S, A, Y, or V |
| 289 | YGGSSGGGGAGX1GX2, wherein X1 and X2 are independently any amino acid, optionally wherein X1 is A, S, Y, or V, optionally wherein X2 is S, A, Y, or V |
| 290 | FGGGSGAGX1GX2, wherein X1 and X2 are independently any amino acid, optionally wherein X1 is A, S, Y, or V, optionally wherein X2 is S, A, Y, or V |
| 291 | CCQSSCCKPSCGAGX1GX2, wherein X1 and X2 are independently any amino acid, optionally wherein X1 is A, S, Y, or V, optionally wherein X2 is S, A, Y, or V |
| 292 | CVSSCCKPQCCGAGX1GX2, wherein X1 and X2 are independently any amino acid, optionally wherein X1 is A, S, Y, or V, optionally wherein X2 is S, A, Y, or V |
| 293 | PITCRRTCYHGAGX1GX2, wherein X1 and X2 are independently any amino acid, optionally wherein X1 is A, S, Y, or V, optionally wherein X2 is S, A, Y, or V |
| 294 | DCKLPCNPCAGAGX1GX2, wherein X1 and X2 are independently any amino acid, optionally wherein X1 is A, S, Y, or V, optionally wherein X2 is S, A, Y, or V |
| 295 | CLPCLPAASCGAGX1GX2, wherein X1 and X2 are independently any amino acid, optionally wherein X1 is A, S, Y, or V, optionally wherein X2 is S, A, Y, or V |
| 296 | CEPAICEPSCGAGX1GX2, wherein X1 and X2 are independently any amino acid, optionally wherein X1 is A, S, Y, or V, optionally wherein X2 is S, A, Y, or V |
| 297 | CQCSCCKPYCSGAGX1GX2, wherein X1 and X2 are independently any amino acid, optionally wherein X1 is A, S, Y, or V, optionally wherein X2 is S, A, Y, or V |
| 298 | FCGFPSCSTSCGAGX1GX2, wherein X1 and X2 are independently any amino acid, optionally wherein X1 is A, S, Y, or V, optionally wherein X2 is S, A, Y, or V |
| 299 | CTPPSCCQLHHAGAGX1GX2, wherein X1 and X2 are independently any amino acid, optionally wherein X1 is A, S, Y, or V, optionally wherein X2 is S, A, Y, or V |
| 300 | SCCAPVYCCKGAGX1GX2, wherein X1 and X2 are independently any amino acid, optionally wherein X1 is A, S, Y, or V, optionally wherein X2 is S, A, Y, or V |
| 301 | GGVCGPSPPCITT(X), wherein X comprises GAGAGS, GAGSGA, GAGAGY, GAGYGA, GAGAGA, GAGAGV, GAGVGA, or GAGAGVGY, or a combination of two or more thereof |
| 302 | GGVCGPSPPC(X), wherein X comprises GAGAGS, GAGSGA, GAGAGY, GAGYGA, GAGAGA, GAGAGV, GAGVGA, or GAGAGVGY, or a combination of two or more thereof |
| 303 | CGPSPPCITT(X), wherein X comprises GAGAGS, GAGSGA, GAGAGY, GAGYGA, GAGAGA, GAGAGV, GAGVGA, or GAGAGVGY, or a combination of two or more thereof |
| 304 | YGGSSGGG(X), wherein X comprises GAGAGS, GAGSGA, GAGAGY, GAGYGA, GAGAGA, GAGAGV, GAGVGA, or GAGAGVGY, or a combination of two or more thereof |
| 305 | FGGGS(X), wherein X comprises GAGAGS, GAGSGA, GAGAGY, GAGYGA, GAGAGA, GAGAGV, GAGVGA, or GAGAGVGY, or a combination of two or more thereof |
| 306 | CCQSSCCKPSC(X), wherein X comprises GAGAGS, GAGSGA, GAGAGY, GAGYGA, GAGAGA, GAGAGV, GAGVGA, or GAGAGVGY, or a combination of two or more thereof |
| 307 | CVSSCCKPQCC(X), wherein X comprises GAGAGS, GAGSGA, GAGAGY, GAGYGA, GAGAGA, GAGAGV, GAGVGA, or GAGAGVGY, or a combination of two or more thereof |
| 308 | PITCRRTCYH(X), wherein X comprises GAGAGS, GAGSGA, GAGAGY, GAGYGA, GAGAGA, GAGAGV, GAGVGA, or GAGAGVGY, or a combination of two or more thereof |
| 309 | DCKLPCNPCA(X), wherein X comprises GAGAGS, GAGSGA, GAGAGY, GAGYGA, GAGAGA, GAGAGV, GAGVGA, or GAGAGVGY, or a combination of two or more thereof |
| 310 | CLPCLPAASC(X), wherein X comprises GAGAGS, GAGSGA, GAGAGY, GAGYGA, GAGAGA, GAGAGV, GAGVGA, or GAGAGVGY, or a combination of two or more thereof |
| 311 | CEPAICEPSC(X), wherein X comprises GAGAGS, GAGSGA, GAGAGY, GAGYGA, GAGAGA, GAGAGV, GAGVGA, or GAGAGVGY, or a combination of two or more thereof |
| 312 | CQCSCCKPYCS(X), wherein X comprises GAGAGS, GAGSGA, GAGAGY, GAGYGA, GAGAGA, GAGAGV, GAGVGA, or GAGAGVGY, or a combination of two or more thereof |
| 313 | FCGFPSCSTSC(X), wherein X comprises GAGAGS, GAGSGA, GAGAGY, GAGYGA, GAGAGA, GAGAGV, GAGVGA, or GAGAGVGY, or a combination of two or more thereof |
| 314 | CTPPSCCQLHHA(X), wherein X comprises GAGAGS, GAGSGA, GAGAGY, GAGYGA, GAGAGA, GAGAGV, GAGVGA, or GAGAGVGY, or a combination of two or more thereof |
| 315 | SCCAPVYCCK(X), wherein X comprises GAGAGS, GAGSGA, GAGAGY, GAGYGA, GAGAGA, GAGAGV, GAGVGA, or GAGAGVGY, or a combination of two or more thereof |

| Collagen/CLP and Keratin Fusion Proteins | |
|---|---|
| 5 | GGVCGPSPPCITTGPTGPTGPAGPRGLQGLQGLQGERGEQGPT |
| 316 | GGVCGPSPPCGPTGPTGPAGPRGLQGLQGLQGERGEQGPT |
| 317 | CGPSPPCITTGPTGPTGPAGPRGLQGLQGLQGERGEQGPT |
| 318 | YGGSSGGGGPTGPTGPAGPRGLQGLQGLQGERGEQGPT |
| 319 | FGGGSGPTGPTGPAGPRGLQGLQGLQGERGEQGPT |
| 320 | CCQSSCCKPSCGPTGPTGPAGPRGLQGLQGLQGERGEQGPT |
| 321 | CVSSCCKPQCCGPTGPTGPAGPRGLQGLQGLQGERGEQGPT |
| 322 | PITCRRTCYHGPTGPTGPAGPRGLQGLQGLQGERGEQGPT |
| 323 | DCKLPCNPCAGPTGPTGPAGPRGLQGLQGLQGERGEQGPT |
| 324 | CLPCLPAASCGPTGPTGPAGPRGLQGLQGLQGERGEQGPT |
| 325 | CEPAICEPSCGPTGPTGPAGPRGLQGLQGLQGERGEQGPT |
| 326 | CQCSCCKPYCSGPTGPTGPAGPRGLQGLQGLQGERGEQGPT |
| 327 | FCGFPSCSTSCGPTGPTGPAGPRGLQGLQGLQGERGEQGPT |
| 328 | CTPPSCCQLHHAGPTGPTGPAGPRGLQGLQGLQGERGEQGPT |
| 329 | SCCAPVYCCKGPTGPTGPAGPRGLQGLQGLQGERGEQGPT |
| 330 | GGVCGPSPPCITTGPTGPT |
| 331 | GGVCGPSPPCGPTGPT |
| 332 | CGPSPPCITTGPTGPT |
| 333 | YGGSSGGGGPTGPT |
| 334 | FGGGSGPTGPT |
| 335 | CCQSSCCKPSCGPTGPT |
| 336 | CVSSCCKPQCCGPTGPT |
| 337 | PITCRRTCYHGPTGPT |
| 338 | DCKLPCNPCAGPTGPT |
| 339 | CLPCLPAASCGPTGPT |
| 340 | CEPAICEPSCGPTGPT |
| 341 | CQCSCCKPYCSGPTGPT |
| 342 | FCGFPSCSTSCGPTGPT |
| 343 | CTPPSCCQLHHAGPTGPT |
| 344 | SCCAPVYCCKGPTGPT |
| 345 | GGVCGPSPPCITTGLQGLQ |
| 346 | GGVCGPSPPCGLQGLQ |
| 347 | CGPSPPCITTGLQGLQ |
| 348 | YGGSSGGGGLQGLQ |
| 349 | FGGGSGLQGLQ |
| 350 | CCQSSCCKPSCGLQGLQ |
| 351 | CVSSCCKPQCCGLQGLQ |
| 352 | PITCRRTCYHGLQGLQ |
| 353 | DCKLPCNPCAGLQGLQ |
| 354 | CLPCLPAASCGLQGLQ |
| 355 | CEPAICEPSCGLQGLQ |
| 356 | CQCSCCKPYCSGLQGLQ |
| 357 | FCGFPSCSTSCGLQGLQ |
| 358 | CTPPSCCQLHHAGLQGLQ |
| 359 | SCCAPVYCCKGLQGLQ |
| 360 | GGVCGPSPPCITT(X), wherein X comprises GXPGXP (each X is independently any amino acid), GZPGZP (each Z is 4-hydroxyproline), or GPAGPA, or a combination of two or more thereof |
| 361 | GGVCGPSPPC(X), wherein X comprises GXPGXP (each X is independently any amino acid), GZPGZP (each Z is 4-hydroxyproline), or GPAGPA, or a combination of two or more thereof |
| 362 | CGPSPPCITT(X), wherein X comprises GXPGXP (each X is independently any amino acid), GZPGZP (each Z is 4-hydroxyproline), or GPAGPA, or a combination of two or more thereof |
| 363 | YGGSSGGG(X), wherein X comprises GXPGXP (each X is independently any amino acid), GZPGZP (each Z is 4-hydroxyproline), or GPAGPA, or a combination of two or more thereof |
| 364 | FGGGS(X), wherein X comprises GXPGXP (each X is independently any amino acid), GZPGZP (each Z is 4-hydroxyproline), or GPAGPA, or a combination of two or more thereof |
| 365 | CCQSSCCKPSC(X), wherein X comprises GXPGXP (each X is independently any amino acid), GZPGZP (each Z is 4-hydroxyproline), or GPAGPA, or a combination of two or more thereof |
| 366 | CVSSCCKPQCC(X), wherein X comprises GXPGXP (each X is independently any amino acid), GZPGZP (each Z is 4-hydroxyproline), or GPAGPA, or a combination of two or more thereof |
| 367 | PITCRRTCYH(X), wherein X comprises GXPGXP (each X is independently any amino acid), GZPGZP (each Z is 4-hydroxyproline), or GPAGPA, or a combination of two or more thereof |
| 368 | DCKLPCNPCA(X), wherein X comprises GXPGXP (each X is independently any amino acid), GZPGZP (each Z is 4-hydroxyproline), or GPAGPA, or a combination of two or more thereof |
| 369 | CLPCLPAASC(X), wherein X comprises GXPGXP (each X is independently any amino acid), GZPGZP (each Z is 4-hydroxyproline), or GPAGPA, or a combination of two or more thereof |
| 370 | CEPAICEPSC(X), wherein X comprises GXPGXP (each X is independently any amino acid), GZPGZP (each Z is 4-hydroxyproline), or GPAGPA, or a combination of two or more thereof |
| 371 | CQCSCCKPYCS(X), wherein X comprises GXPGXP (each X is independently any amino acid), GZPGZP (each Z is 4-hydroxyproline), or GPAGPA, or a combination of two or more thereof |
| 372 | FCGFPSCSTSC(X), wherein X comprises GXPGXP (each X is independently any amino acid), GZPGZP (each Z is 4-hydroxyproline), or GPAGPA, or a combination of two or more thereof |
| 373 | CTPPSCCQLHHA(X), wherein X comprises GXPGXP (each X is independently any amino acid), GZPGZP (each Z is 4-hydroxyproline), or GPAGPA, or a combination of two or more thereof |
| 374 | SCCAPVYCCK(X), wherein X comprises GXPGXP (each X is independently any amino acid), GZPGZP (each Z is 4-hydroxyproline), or GPAGPA, or a combination of two or more thereof |

| Resilin/RLP and Keratin Fusion Proteins | |
|---|---|
| 4 | GGVCGPSPPCITTGGRPSDSYGAPGGGN |
| 375 | GGVCGPSPPCGGRPSDSYGAPGGGN |
| 376 | CGPSPPCITTGGRPSDSYGAPGGGN |
| 377 | YGGSSGGGGGRPSDSYGAPGGGN |
| 378 | FGGGSGGRPSDSYGAPGGGN |
| 379 | CCQSSCCKPSCGGRPSDSYGAPGGGN |
| 380 | CVSSCCKPQCCGGRPSDSYGAPGGGN |
| 381 | PITCRRTCYHGGRPSDSYGAPGGGN |
| 382 | DCKLPCNPCAGGRPSDSYGAPGGGN |
| 383 | CLPCLPAASCGGRPSDSYGAPGGGN |
| 384 | CEPAICEPSCGGRPSDSYGAPGGGN |
| 385 | CQCSCCKPYCSGGRPSDSYGAPGGGN |
| 386 | FCGFPSCSTSCGGRPSDSYGAPGGGN |
| 387 | CTPPSCCQLHHAGGRPSDSYGAPGGGN |
| 388 | SCCAPVYCCKGGRPSDSYGAPGGGN |
| 389 | GGVCGPSPPCITTGAPAQTPSSQY |
| 390 | GGVCGPSPPCGAPAQTPSSQY |
| 391 | CGPSPPCITTGAPAQTPSSQY |
| 392 | YGGSSGGGGAPAQTPSSQY |
| 393 | FGGGSGAPAQTPSSQY |
| 394 | CCQSSCCKPSCGAPAQTPSSQY |
| 395 | CVSSCCKPQCCGAPAQTPSSQY |
| 396 | PITCRRTCYHGAPAQTPSSQY |
| 397 | DCKLPCNPCAGAPAQTPSSQY |
| 398 | CLPCLPAASCGAPAQTPSSQY |
| 399 | CEPAICEPSCGAPAQTPSSQY |
| 400 | CQCSCCKPYCSGAPAQTPSSQY |
| 401 | FCGFPSCSTSCGAPAQTPSSQY |
| 402 | CTPPSCCQLHHAGAPAQTPSSQY |
| 403 | SCCAPVYCCKGAPAQTPSSQY |
| 404 | GGVCGPSPPCITTAQTPSSQYGAP |
| 405 | GGVCGPSPPCAQTPSSQYGAP |
| 406 | CGPSPPCITTAQTPSSQYGAP |
| 407 | YGGSSGGGAQTPSSQYGAP |
| 408 | FGGGSAQTPSSQYGAP |
| 409 | CCQSSCCKPSCAQTPSSQYGAP |
| 410 | CVSSCCKPQCCAQTPSSQYGAP |
| 411 | PITCRRTCYHAQTPSSQYGAP |
| 412 | DCKLPCNPCAAQTPSSQYGAP |
| 413 | CLPCLPAASCAQTPSSQYGAP |
| 414 | CEPAICEPSCAQTPSSQYGAP |
| 415 | CQCSCCKPYCSAQTPSSQYGAP |
| 416 | FCG FPSCSTSCAQTPSSQYGAP |
| 417 | CTPPSCCQLHHAAQTPSSQYGAP |
| 418 | SCCAPVYCCKAQTPSSQYGAP |
| Abductin/ALP and Keratin Fusion Proteins | |
| 6 | GGVCGPSPPCITTGGFGGMGGGSGGFGGMGGGSGGFGGMGGGS |
| 419 | GGVCGPSPPCGGFGGMGGGSGGFGGMGGGSGGFGGMGGGS |
| 420 | CGPSPPCITTGGFGGMGGGSGGFGGMGGGSGGFGGMGGGS |
| 421 | YGGSSGGGGGFGGMGGGSGGFGGMGGGSGGFGGMGGGS |
| 422 | FGGGSGGFGGMGGGSGGFGGMGGGSGGFGGMGGGS |
| 423 | CCQSSCCKPSCGGFGGMGGGSGGFGGMGGGSGGFGGMGGGS |
| 424 | CVSSCCKPQCCGGFGGMGGGSGGFGGMGGGSGGFGGMGGGS |
| 425 | PITCRRTCYHGGFGGMGGGSGGFGGMGGGSGGFGGMGGGS |
| 426 | DCKLPCNPCAGGFGGMGGGSGGFGGMGGGSGGFGGMGGGS |
| 427 | CLPCLPAASCGGFGGMGGGSGGFGGMGGGSGGFGGMGGGS |
| 428 | CEPAICEPSCGGFGGMGGGSGGFGGMGGGSGGFGGMGGGS |
| 429 | CQCSCCKPYCSGGFGGMGGGSGGFGGMGGGSGGFGGMGGGS |
| 430 | FCGFPSCSTSCGGFGGMGGGSGGFGGMGGGSGGFGGMGGGS |
| 431 | CTPPSCCQLHHAGGFGGMGGGSGGFGGMGGGSGGFGGMGGGS |
| 432 | SCCAPVYCCKGGFGGMGGGSGGFGGMGGGSGGFGGMGGGS |
| 433 | GGVCGPSPPCITTMGGG |
| 434 | GGVCGPSPPCMGGG |
| 435 | CGPSPPCITTMGGG |
| 436 | YGGSSGGGMGGG |
| 437 | FGGGSMGGG |
| 438 | CCQSSCCKPSCMGGG |
| 439 | CVSSCCKPQCCMGGG |
| 440 | PITCRRTCYHMGGG |
| 441 | DCKLPCNPCAMGGG |
| 442 | CLPCLPAASCMGGG |
| 443 | CEPAICEPSCMGGG |
| 444 | CQCSCCKPYCSMGGG |
| 445 | FCGFPSCSTSCMGGG |
| 446 | CTPPSCCQLHHAMGGG |
| 447 | SCCAPVYCCKMGGG |
| 448 | GGVCGPSPPCITTFGGMG |
| 449 | GGVCGPSPPCFGGMG |
| 450 | CGPSPPCITTFGGMG |
| 451 | YGGSSGGGFGGMG |
| 452 | FGGGSFGGMG |
| 453 | CCQSSCCKPSCFGGMG |
| 454 | CVSSCCKPQCCFGGMG |
| 455 | PITCRRTCYHFGGMG |
| 456 | DCKLPCNPCAFGGMG |
| 457 | CLPCLPAASCFGGMG |
| 458 | CEPAICEPSCFGGMG |
| 459 | CQCSCCKPYCSFGGMG |
| 460 | FCGFPSCSTSCFGGMG |
| 461 | CTPPSCCQLHHAFGGMG |
| 462 | SCCAPVYCCKFGGMG |
| 463 | GGVCGPSPPCITT(X), wherein X comprises GGFGGMGGGS, FGGMGGG, GGFGGMGGG, or FGGMGGGNAG, or a combination of two or more thereof |
| 464 | GGVCGPSPPC(X), wherein X comprises GGFGGMGGGS, FGGMGGG, GGFGGMGGG, or FGGMGGGNAG, or a combination of two or more thereof |
| 465 | CGPSPPCITT(X), wherein X comprises GGFGGMGGGS, FGGMGGG, GGFGGMGGG, or FGGMGGGNAG, or a combination of two or more thereof |
| 466 | YGGSSGGG(X), wherein X comprises GGFGGMGGGS, FGGMGGG, GGFGGMGGG, or FGGMGGGNAG, or a combination of two or more thereof |
| 467 | FGGGS(X), wherein X comprises GGFGGMGGGS, FGGMGGG, GGFGGMGGG, or FGGMGGGNAG, or a combination of two or more thereof |
| 468 | CCQSSCCKPSC(X), wherein X comprises GGFGGMGGGS, FGGMGGG, GGFGGMGGG, or FGGMGGGNAG, or a combination of two or more thereof |
| 469 | CVSSCCKPQCC(X), wherein X comprises GGFGGMGGGS, FGGMGGG, GGFGGMGGG, or FGGMGGGNAG, or a combination of two or more thereof |
| 470 | PITCRRTCYH(X), wherein X comprises GGFGGMGGGS, FGGMGGG, GGFGGMGGG, or FGGMGGGNAG, or a combination of two or more thereof |
| 471 | DCKLPCNPCA(X), wherein X comprises GGFGGMGGGS, FGGMGGG, GGFGGMGGG, or FGGMGGGNAG, or a combination of two or more thereof |
| 472 | CLPCLPAASC(X), wherein X comprises GGFGGMGGGS, FGGMGGG, GGFGGMGGG, or FGGMGGGNAG, or a combination of two or more thereof |
| 473 | CEPAICEPSC(X), wherein X comprises GGFGGMGGGS, FGGMGGG, GGFGGMGGG, or FGGMGGGNAG, or a combination of two or more thereof |
| 474 | CQCSCCKPYCS(X), wherein X comprises GGFGGMGGGS, FGGMGGG, GGFGGMGGG, or FGGMGGGNAG, or a combination of two or more thereof |
| 475 | FCGFPSCSTSC(X), wherein X comprises GGFGGMGGGS, FGGMGGG, GGFGGMGGG, or FGGMGGGNAG, or a combination of two or more thereof |
| 476 | CTPPSCCQLHHA(X), wherein X comprises GGFGGMGGGS, FGGMGGG, GGFGGMGGG, or FGGMGGGNAG, or a combination of two or more thereof |
| 477 | SCCAPVYCCK(X), wherein X comprises GGFGGMGGGS, FGGMGGG, GGFGGMGGG, or FGGMGGGNAG, or a combination of two or more thereof |

| Linker/Spacer-like and Keratin Fusion Proteins | |
|---|---|
| 44 | GGVCGPSPPCITTGQGQGQGQGQGQGQGQGQGQGQGQGQGQGQ |
| 45 | GGVCGPSPPCITTAKAKAKAKAKAKAKAKAKAKAKAKAKAKAK |
| 46 | GGVCGPSPPCITTLKLKLKLKLKLKLKLKLKLKLKLKLKLKLK |
| 47 | GGVCGPSPPCITTGAGAGAGAGAGAGAGAGAGAGAGAGAGAGA |
| 478 | GGVCGPSPPCGQGQGQGQGQGQGQGQGQGQGQGQGQGQGQ |
| 479 | CGPSPPCITTGQGQGQGQGQGQGQGQGQGQGQGQGQGQGQ |
| 480 | YGGSSGGGGQGQGQGQGQGQGQGQGQGQGQGQGQGQGQ |
| 481 | FGGGSGQGQGQGQGQGQGQGQGQGQGQGQGQGQGQ |
| 482 | CCQSSCCKPSCGQGQGQGQGQGQGQGQGQGQGQGQGQGQGQ |
| 483 | CVSSCCKPQCCGQGQGQGQGQGQGQGQGQGQGQGQGQGQGQ |
| 484 | PITCRRTCYHGQGQGQGQGQGQGQGQGQGQGQGQGQGQGQ |
| 485 | DCKLPCNPCAGQGQGQGQGQGQGQGQGQGQGQGQGQGQGQ |
| 486 | CLPCLPAASCGQGQGQGQGQGQGQGQGQGQGQGQGQGQGQ |
| 487 | CEPAICEPSCGQGQGQGQGQGQGQGQGQGQGQGQGQGQGQ |
| 488 | CQCSCCKPYCSGQGQGQGQGQGQGQGQGQGQGQGQGQGQGQ |
| 489 | FCGFPSCSTSCGQGQGQGQGQGQGQGQGQGQGQGQGQGQGQ |
| 490 | CTPPSCCQLHHAGQGQGQGQGQGQGQGQGQGQGQGQGQGQGQ |
| 491 | SCCAPVYCCKGQGQGQGQGQGQGQGQGQGQGQGQGQGQGQ |
| 492 | GGVCGPSPPCAKAKAKAKAKAKAKAKAKAKAKAKAKAKAK |
| 493 | CGPSPPCITTAKAKAKAKAKAKAKAKAKAKAKAKAKAKAK |
| 494 | YGGSSGGGAKAKAKAKAKAKAKAKAKAKAKAKAKAKAK |
| 495 | FGGGSAKAKAKAKAKAKAKAKAKAKAKAKAKAKAK |
| 496 | CCQSSCCKPSCAKAKAKAKAKAKAKAKAKAKAKAKAKAKAK |
| 497 | CVSSCCKPQCCAKAKAKAKAKAKAKAKAKAKAKAKAKAKAK |
| 498 | PITCRRTCYHAKAKAKAKAKAKAKAKAKAKAKAKAKAKAK |
| 499 | DCKLPCNPCAAKAKAKAKAKAKAKAKAKAKAKAKAKAKAK |
| 500 | CLPCLPAASCAKAKAKAKAKAKAKAKAKAKAKAKAKAKAK |
| 501 | CEPAICEPSCAKAKAKAKAKAKAKAKAKAKAKAKAKAKAK |
| 502 | CQCSCCKPYCSAKAKAKAKAKAKAKAKAKAKAKAKAKAKAK |
| 503 | FCGFPSCSTSCAKAKAKAKAKAKAKAKAKAKAKAKAKAKAK |
| 504 | CTPPSCCQLHHAAKAKAKAKAKAKAKAKAKAKAKAKAKAKAK |
| 505 | SCCAPVYCCKAKAKAKAKAKAKAKAKAKAKAKAKAKAKAK |
| 506 | GGVCGPSPPCLKLKLKLKLKLKLKLKLKLKLKLKLKLKLK |
| 507 | CGPSPPCITTLKLKLKLKLKLKLKLKLKLKLKLKLKLKLK |
| 508 | YGGSSGGGLKLKLKLKLKLKLKLKLKLKLKLKLKLKLK |
| 509 | FGGGSLKLKLKLKLKLKLKLKLKLKLKLKLKLKLK |
| 510 | CCQSSCCKPSCLKLKLKLKLKLKLKLKLKLKLKLKLKLKLK |
| 511 | CVSSCCKPQCCLKLKLKLKLKLKLKLKLKLKLKLKLKLKLK |
| 512 | PITCRRTCYHLKLKLKLKLKLKLKLKLKLKLKLKLKLKLK |
| 513 | DCKLPCNPCALKLKLKLKLKLKLKLKLKLKLKLKLKLKLK |
| 514 | CLPCLPAASCLKLKLKLKLKLKLKLKLKLKLKLKLKLKLK |
| 515 | CEPAICEPSCLKLKLKLKLKLKLKLKLKLKLKLKLKLKLK |
| 516 | CQCSCCKPYCSLKLKLKLKLKLKLKLKLKLKLKLKLKLKLK |
| 517 | FCGFPSCSTSCLKLKLKLKLKLKLKLKLKLKLKLKLKLKLK |
| 518 | CTPPSCCQLH HALKLKLKLKLKLKLKLKLKLKLKLKLKLKLK |
| 519 | SCCAPVYCCKLKLKLKLKLKLKLKLKLKLKLKLKLKLKLK |
| 520 | GGVCGPSPPCGAGAGAGAGAGAGAGAGAGAGAGAGAGAGA |
| 521 | CGPSPPCITTGAGAGAGAGAGAGAGAGAGAGAGAGAGAGA |
| 522 | YGGSSGGGGAGAGAGAGAGAGAGAGAGAGAGAGAGAGA |
| 523 | FGGGSGAGAGAGAGAGAGAGAGAGAGAGAGAGAGA |
| 524 | CCQSSCCKPSCGAGAGAGAGAGAGAGAGAGAGAGAGAGAGA |
| 525 | CVSSCCKPQCCGAGAGAGAGAGAGAGAGAGAGAGAGAGAGA |
| 526 | PITCRRTCYHGAGAGAGAGAGAGAGAGAGAGAGAGAGAGA |
| 527 | DCKLPCNPCAGAGAGAGAGAGAGAGAGAGAGAGAGAGAGA |
| 528 | CLPCLPAASCGAGAGAGAGAGAGAGAGAGAGAGAGAGAGA |
| 529 | CEPAICEPSCGAGAGAGAGAGAGAGAGAGAGAGAGAGAGA |
| 530 | CQCSCCKPYCSGAGAGAGAGAGAGAGAGAGAGAGAGAGAGA |
| 531 | FCGFPSCSTSCGAGAGAGAGAGAGAGAGAGAGAGAGAGAGA |
| 532 | CTPPSCCQLHHAGAGAGAGAGAGAGAGAGAGAGAGAGAGAGA |
| 533 | SCCAPVYCCKGAGAGAGAGAGAGAGAGAGAGAGAGAGAGA |
| 534 | GGVCGPSPPCITT(X), wherein X comprises (GQ)n, where n is 1-20; (AK)n, where n is 1-20; (LK)n, where n is 1-20; or (GA)n, where n is 1-20; or a combination of two or more thereof |
| 535 | GGVCGPSPPC(X), wherein X comprises (GQ)n, where n is 1-20; (AK)n, where n is 1-20; (LK)n, where n is 1-20; or (GA)n, where n is 1-20; or a combination of two or more thereof |
| 536 | CGPSPPCITT(X), wherein X comprises (GQ)n, where n is 1-20; (AK)n, where n is 1-20; (LK)n, where n is 1-20; or (GA)n, where n is 1-20; or a combination of two or more thereof |
| 537 | YGGSSGGG(X), wherein X comprises (GQ)n, where n is 1-20; (AK)n, where n is 1-20; (LK)n, where n is 1-20; or (GA)n, where n is 1-20; or a combination of two or more thereof |
| 538 | FGGGS(X), wherein X comprises (GQ)n, where n is 1-20; (AK)n, where n is 1-20; (LK)n, where n is 1-20; or (GA)n, where n is 1-20; or a combination of two or more thereof |
| 539 | CCQSSCCKPSC(X), wherein X comprises (GQ)n, where n is 1-20; (AK)n, where n is 1-20; (LK)n, where n is 1-20; or (GA)n, where n is 1-20; or a combination of two or more thereof |
| 540 | CVSSCCKPQCC(X), wherein X comprises (GQ)n, where n is 1-20; (AK)n, where n is 1-20; (LK)n, where n is 1-20; or (GA)n, where n is 1-20; or a combination of two or more thereof |
| 541 | PITCRRTCYH(X), wherein X comprises (GQ)n, where n is 1-20; (AK)n, where n is 1-20; (LK)n, where n is 1-20; or (GA)n, where n is 1-20; or a combination of two or more thereof |
| 542 | DCKLPCNPCA(X), wherein X comprises (GQ)n, where n is 1-20; (AK)n, where n is 1-20; (LK)n, where n is 1-20; or (GA)n, where n is 1-20; or a combination of two or more thereof |
| 543 | CLPCLPAASC(X), wherein X comprises (GQ)n, where n is 1-20; (AK)n, where n is 1-20; (LK)n, where n is 1-20; or (GA)n, where n is 1-20; or a combination of two or more thereof |
| 544 | CEPAICEPSC(X), wherein X comprises (GQ)n, where n is 1-20; (AK)n, where n is 1-20; (LK)n, where n is 1-20; or (GA)n, where n is 1-20; or a combination of two or more thereof |
| 545 | CQCSCCKPYCS(X), wherein X comprises (GQ)n, where n is 1-20; (AK)n, where n is 1-20; (LK)n, where n is 1-20; or (GA)n, where n is 1-20; or a combination of two or more thereof |
| 546 | FCGFPSCSTSC(X), wherein X comprises (GQ)n, where n is 1-20; (AK)n, where n is 1-20; (LK)n, where n is 1-20; or (GA)n, where n is 1-20; or a combination of two or more thereof |
| 547 | CTPPSCCQLHHA(X), wherein X comprises (GQ)n, where n is 1-20; (AK)n, where n is 1-20; (LK)n, where n is 1-20; or (GA)n, where n is 1-20; or a combination of two or more thereof |
| 548 | SCCAPVYCCK(X), wherein X comprises (GQ)n, where n is 1-20; (AK)n, where n is 1-20; (LK)n, where n is 1-20; or (GA)n, where n is 1-20; or a combination of two or more thereof |

| Additional Fusion Proteins | |
|---|---|
| 24 | GGVCGPSPPCITTGAGAGSGAGAGSVPAVGVPAVGVPAVG |
| 550 | GGVCGPSPPCITTRGAGAGSGAGAGSGAGAGSVPAVGVPAVGVPAVGVPAVGVPAVGVPAVGVPA VGVPAVGVPAVGVPAVG |
| 551 | GGRPSDSYGAPGGGNGGRPSDSYGAPGGGNGGRPSDSYGAPGGGNGGRPSDSYGAPGGGNGGRP SDSYGAPGGGNGGRPSDSYGAPGGGNGGRPSDSYGAPGGGNGGRPSDSYGAPGGGNGGRPSDSYG APGGGNGGRPSDSYGAPGGGNGQGQGQGQGQGQGQGQGQGGVCGPSPPCITTGQGQGQG QGQGQGQGQGQGQVPAVGVPAVGVPAVGVPAVGVPAVGVPAVGVPAVGVPAVGVPAVGVPAVG GQGQGQGQGQGQGQGQGQGQGGVCGPSPPCITTGQGQGQGQGQGQGQGQGQGGRPSDSY GAPGGGNGGRPSDSYGAPGGGNGGRPSDSYGAPGGGNGGRPSDSYGAPGGGNGGRPSDSYGAPG GGNGGRPSDSYGAPGGGNGGRPSDSYGAPGGGNGGRPSDSYGAPGGGNGGRPSDSYGAPGGGNG GRPSDSYGAPGGGN |

| Opioid peptides | |
|---|---|
| 552 | YGGFL |
| 553 | YPWF |
| 554 | YGGFM |
| 555 | YPFF |

| Additional relevant peptides | |
|---|---|
| 556 | EEMQRR |
| 557 | MGWCTASVPPQCYG |
| 558 | ATAKAWLKGIGAGAGAGA |
| 559 | RRQMEEGAGAGAGA |
| 560 | GGVCGPSPPCITTATAKAWLKGI |
| 561 | GGVCGPSPPCITTGAGAGAGATAKAWLKGI |
| 562 | ATAKAWLKGIGGVCGPSPPCITT |
| 563 | ATAKAWLKGIAGAGAGAGGVCGPSPPCITT |
| 564 | GGVCGPSPPCITTATAKAWLKGIRRQMEE |
| 565 | RRQMEEATAKAWLKGIGGVCGPSPPCITT |
| 566 | GGVCGPSPPCITTRRQMEE |
| 567 | GVCGPSPPCITTGAGAGAGARRQMEE |
| 568 | RRQMEEGAGAGAGAGGVCGPSPPCITT |
| 569 | ATAKAWLKGIMGWCTASVPPQCYG |
| 570 | RRQMEEMGWCTASVPPQCYG |
| 571 | YGRKKRRQRRR |
| 572 | GGVCGPSPPCITTGTCYINDDEGCTKRCQGIYIYRREAVMGHC |
| 573 | GGVCGPSPPCITTRCAERCSEASIQDRCLKYCGICCEK |
| 574 | GGVCGPSPPCITTGTYGNKDECPCYRDMKNSKGKGKCP |
| 575 | GRKKRRQRRRPPQ |
| 576 | RQIKIWFQNRRMKWKK |
| 577 | RIMRILRILKLAR |
| 578 | LLIILRRRRIRKQAHAHSK |
| 579 | GVCGPSPPCITTDRDDQAAWFSQY |
| 580 | MVYYPNPCSPYPCRNGGTCKKRGLYSYKCYCRKGYTGKNCQYNACFPNPCLNGGTCGYVYGYPYYKCS CPYGYYGKQCQLKKYGGVCGPSPPCITT |
| 581 | GGVCGPSPPCITTERSRSSDGKSSSQVNRSRHENTSQVPLQESRTRKRRGSRVSQDRDSEGH |
| 582 | GGVCGPSPPCITTDGTRHSGSRHHEASSQADSSRHSQVGQGQSSGPRTSRNQGSSVSQDSDSQGH |
| 583 | ERSRSSDGKSSSQVNRSRHENTSQVPLQESRTRKRRGSRVSQDRDSEGHGGVCGPSPPCITTDGTRHS GSRHHEASSQADSSRHSQVGQGQSSGPRTSRNQGSSVSQDSDSQGH |

The term "comprising" whenever used in this document is intended to indicate the presence of stated features, integers, steps, components, but not to preclude the presence or addition of one or more other features, integers, steps, components or groups thereof.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (over the whole the sequence) alignment of two sequences that maximizes the number of matches and minimizes the number of gaps. The BLAST algorithm (Altschul et al.

(1990) J Mol Biol 215: 403-10) calculates percent sequence identity and performs a statistical analysis of the similarity between the two sequences. The software for performing BLAST analysis is publicly available through the National Centre for Biotechnology Information (NCBI). Global percentages of similarity and identity may also be determined using one of the methods available in the MatGAT software package (Campanella et al., BMC Bioinformatics. 2003 Jul 10; 4:29. MatGAT: an application that generates similarity/identity matrices using protein or DNA sequences). Minor manual editing may be performed to optimise alignment between conserved motifs, as would be apparent to a person skilled in the art. The sequence identity values, which are indicated in the present subject matter as a percentage were determined over the entire amino acid sequence, using BLAST with the default parameters.

As used in the specification and claims, the singular forms "a", "an" and "the" include plural references unless the context clearly dictates otherwise. For example, the term "a sample" includes a plurality of samples, including mixtures thereof.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

The terms "determining," "measuring," "evaluating," "assessing," "assaying," and "analyzing" are often used interchangeably herein to refer to forms of measurement. The terms include determining if an element is present or not (for example, detection). These terms can include quantitative, qualitative or quantitative and qualitative determinations. Assessing can be relative or absolute. "Detecting the presence of" can include determining the amount of something present in addition to determining whether it is present or absent depending on the context.

As used herein, the term "about" a number refers to that number plus or minus 10% of that number. The term "about" a range refers to that range minus 10% of its lowest value and plus 10% of its greatest value.

As used herein, the terms "pharmaceutically acceptable" and "cosmetically acceptable" are used interchangeably and refer to those compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio. More specifically, pharmaceutically acceptable refers to a material, compound, or composition which is suitable for use in contact with the skin, scalp, or hair. Pharmaceutically acceptable materials are known to those of ordinary skill in the art.

As used herein, the terms "treatment" or "treating" are used in reference to a pharmaceutical or other intervention regimen for obtaining beneficial or desired results in the recipient. Beneficial or desired results include but are not limited to a therapeutic benefit and/or a prophylactic benefit. A therapeutic benefit may refer to eradication or amelioration of symptoms or of an underlying disorder being treated. Also, a therapeutic benefit can be achieved with the eradication or amelioration of one or more of the physiological symptoms associated with the underlying disorder such that an improvement is observed in the subject, notwithstanding that the subject may still be afflicted with the underlying disorder. A prophylactic effect includes delaying, preventing, or eliminating the appearance of a disease or condition, delaying or eliminating the onset of symptoms of a disease or condition, slowing, halting, or reversing the progression of a disease or condition, or any combination thereof. For prophylactic benefit, a subject at risk of developing a particular disease, or to a subject reporting one or more of the physiological symptoms of a disease may undergo treatment, even though a diagnosis of this disease may not have been made.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Furthermore, it is to be understood that the invention encompasses all variations, combinations, and permutations in which one or more limitations, elements, clauses, descriptive terms, etc., from one or more of the claims or from relevant portions of the description is introduced into another claim. For example, any claim that is dependent on another claim can be modified to include one or more limitations found in any other claim that is dependent on the same base claim.

Furthermore, where the claims recite a composition, it is to be understood that methods of using the composition for any of the purposes disclosed herein are included, and methods of making the composition according to any of the methods of making disclosed herein or other methods known in the art are included, unless otherwise indicated or unless it would be evident to one of ordinary skill in the art that a contradiction or inconsistency would arise.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

The disclosure should not be seen in any way restricted to the embodiments described and a person with ordinary skill in the art will foresee many possibilities to modifications thereof.

The above-described embodiments are combinable.

The following claims further set out particular embodiments of the disclosure.

## Claims

1. Stable emulsified cosmetic composition, comprising:
a bioactive mixture; a phospholipid as an emulsifier; and di-alcohol as a solvent;
wherein the bioactive mixture comprises a natural deep eutectic solvent and an active cosmetic compound;
wherein the natural deep eutectic solvent is selected from a list consisting of a mixture of at least two of: lactic acid, glycolic acid, decanoic acid, citric acid, maleic acid, malic acid, oxalic acid, tartaric acid, oleic acid, palmitic acid, enanthic acid,
formic acid, caprylic acid, ethylene glycol, propylene glycol, glycine, glycerol, glucose, transcutol, menthol, sodium lactate, sodium acetate, sodium citrate, xylitol and sorbitol more preferably lactic acid, glycolic acid, glycine, glycerol, ethylene glycol and propylene glycol.

2. Cosmetic composition according to the previous claim comprising:
0.01 to 30% (w/w) of the bioactive mixture, preferably 10 to 30% (w/w), more preferably 15 to 20% (w/w);
0.01 to 50% (w/w) of the phospholipid as an emulsifier, preferably 10 to 50% (w/w), more preferably 20 to 50% (w/w), even more preferably 25 to 40% (w/w);
0.01 to 40% (w/w) of the di-alcohol as a solvent, preferably 10 to 40% (w/w), more preferably 20 to 30% (w/w).

3. Cosmetic composition according to any of the previous claims wherein the phospholipid is selected from a list consisting of: phosphatidylcholine, phosphatidylethanolamine, phosphatidylinositol, phosphatidylserine, phosphatidic acid, lecithin including unsaturated as well as hydrogenated phospholipids, or mixtures thereof; preferably phosphatidylcholine, phosphatidylethanolamine, phosphatidylinositol, lecithin or mixtures thereof; lecithin more preferably phosphatidylcholine and /or lecithin.

4. Cosmetic composition according to any of the previous claims wherein at least two components of the natural deep eutectic solvent mixture are glycerol and glycolic acid or lactic acid and glycerol.

5. Cosmetic composition according to any of the previous claims wherein the molar ratio between the two components of NADES ranges from 20:1 - 1:20; preferably 10:1 to 1:10, more preferably 1:2 to 1:4.

6. Cosmetic composition according to any of the previous claims wherein the active cosmetic compound is selected from: a peptide, icilin, menthol, carboxylated icilin, carboxylated menthol, baicalin, curcumin, caffeine, or mixtures thereof; preferably wherein the amount of the active cosmetic compound ranges from 0.01 to 10 % (w/w), more from preferably 0.01 to 5 % (w/w), even more preferably from 0.5 to 2 % (w/w).

7. Cosmetic composition according to any of the previous claims wherein the di-alcohol is selected from a list consisting of : 1,2-Propanediol; 1,3-Propanediol; 1,2-Butanediol; 1,3-Butanediol; 1,4-Butanediol; 2,3-Butanediol; 1,2-Hexanediol; 1,6-Hexanodiol; 1,5-Pentanediol; 1,2-Octanediol; 1,8-Octanediol; 1,2-Decanediol; 1,10-Decanediol; Methylpropanediol; 2-Butyl-2-ethyl-1,3-propanediol, Isopentyldiol and Ethane-1,2-diol or mixtures thereof; preferably 1,2-Propanediol; 1,3-Propanediol; 1,2-Butanediol; 1,3-Butanediol; 1,4-Butanediol; 2,3-Butanediol; 1,2-Hexanediol; 1,6-Hexanodiol, or mixture thereof; more preferably 1,3-Propanediol, 1,2-Butanediol and 1,2-Hexanediol; or mixture thereof.

8. Cosmetic composition according to any of the previous claims further comprising a natural oil, a propellant, a fragrance, an oil, or mixture thereof; preferably an essential oil, an insect-based oil, a plant-based oil, or mixtures thereof.

9. Cosmetic composition according to any of the previous claims further comprising a peptide, preferably wherein the peptide is at least a peptide 80%, 85%, 90%, 91%, 92%, 93%, 94%, 95%, 96%, 97%, 98%, 99%, or 100% sequence identity to a sequence selected from a list consisting of:
SEQ ID No 552: YGGFL;
SEQ ID No 553: YPWF;
SEQ ID No 554: YGGFM;
SEQ ID No 555: YPFF;
SEQ ID No 556: EEMQRR;
SEQ ID No 557: MGWCTASVPPQCYG;
SEQ ID No 558: ATAKAWLKGIGAGAGAGA;
SEQ ID No 559: RRQMEEGAGAGAGA;
SEQ ID No 560: GGVCGPSPPCITTATAKAWLKGI;
SEQ ID No 561: GGVCGPSPPCITTGAGAGAGATAKAWLKGI;
SEQ ID No 562: ATAKAWLKGIGGVCGPSPPCITT;
SEQ ID No 563: ATAKAWLKGIAGAGAGAGGVCGPSPPCITT;
SEQ ID No 564: GGVCGPSPPCITTATAKAWLKGIRRQMEE;
SEQ ID No 565: RRQMEEATAKAWLKGIGGVCGPSPPCITT;
SEQ ID No 566: GGVCGPSPPCITTRRQMEE;
SEQ ID No 584: GGVCGPSPPCITTGAGAGAGARRQMEE;
SEQ ID No 585: RRQMEEGGVCGPSPPCITT;
SEQ ID No 586: RRQMEEGGVCGPSPPCITTGRKKRRQRRRPPQ;
SEQ ID No 587: GRKKRRQRRRPPQGGVCGPSPPCITTRRQMEE;
SEQ ID No 568: RRQMEEGAGAGAGAGGVCGPSPPCITT;
SEQ ID No 569: ATAKAWLKGIMGWCTASVPPQCYG;
SEQ ID No 570: RRQMEEMGWCTASVPPQCYG;
SEQ ID No 2: GGVCGPSPPCITTVPGVGVPGVGVPGVGVPGVGVPGVGVPGVG;
SEQ ID No 549: GGVCGPSPPCITTGAGAGSGAGAGSVPAVGVPAVGVPAVG;
SEQ ID No 5: GGVCGPSPPCITTGPTGPTGPAGPRGLQGLQGLQGERGEQGPT;
SEQ ID No 4: GGVCGPSPPCITTGGRPSDSYGAPGGGN;
SEQ ID No 6: GGVCGPSPPCITTGGFGGMGGGSGGFGGMGGGSGGFGGMGGGS;
SEQ ID No 45: GGVCGPSPPCITTAKAKAKAKAKAKAKAKAKAKAKAKAKAKAK;
SEQ ID No 46: GGVCGPSPPCITTLKLKLKLKLKLKLKLKLKLKLKLKLKLKLK;
SEQ ID No 573: GGVCGPSPPCITTRCAERCSEASIQDRCLKYCGICCEK;
SEQ ID No 574: GGVCGPSPPCITTGTYGNKDECPCYRDMKNSKGKGKCP;
SEQ ID No 572: GGVCGPSPPCITTGTCYINDDEGCTKRCQGIYIYRREAVMGHC;
SEQ ID No 588: LKLKLKLKLKLKLKLKLKLK;
SEQ ID No 589: LELELELELELELELELELE;
SEQ ID No 571: YGRKKRRQRRR;
SEQ ID No 575: GRKKRRQRRRPPQ;
SEQ ID No 576: RQIKIWFQNRRMKWKK;
SEQ ID No 590: RKKNPNCRRH;
SEQ ID No 577: RIMRILRILKLAR;
SEQ ID No 578: LLIILRRRRIRKQAHAHSK;
SEQ ID No 579: GVCGPSPPCITTDRDDQAAWFSQY;
SEQ ID No 580: 
MVYYPNPCSPYPCRNGGTCKKRGLYSYKCYCRKGYTGKNCQYNACFPNPCLNGGTCGYVYG
YPYYKCSCPYGYYGKQCQLKKYGGVCGPSPPCITT;
SEQ ID No 581: GGVCG PSPPCITTERSRSSDG KSSSQVN RSRH E NTSQVPLQESRTRKRRGSRVSQD RDSEG H;
SEQ ID No 582: 
GGVCGPSPPCITTDGTRHSGSRHHEASSQADSSRHSQVGQGQSSGPRTSRNQGSSVSQDSD
SQGH;
SEQ ID No 583: 
ERSRSSDGKSSSQVNRSRHENTSQVPLQESRTRKRRGSRVSQDRDSEGHGGVCGPSPPCITT
DGTRHSGSRHHEASSQADSSRHSQVGQGQSSGPRTSRNQGSSVSQDSDSQGH,
SEQ ID No 46: GGVCGPSPPCITT; or sequence of table 2 or 3;
or mixtures thereof.

10. Cosmetic composition according to any of the previous claims, wherein the amount peptide ranges from 0.0001 % to 20 % (w/w); preferably the amount peptide ranges from 0.001% to 5 % (w/w); more preferably from 0.1% to about 1% (w/w).

11. Cosmetic composition according to any of the previous claims, wherein the composition does not contain water.

12. Cosmetic composition according to any of the previous claims, comprising at least one excipient suitable for dermatological use; preferably wherein the at least one excipient suitable for dermatological use is selected from the following list: preservative, thickener, organic polymer, humectant, silicone, oil, fragrance, vitamin, buffer, antimicrobial agent, antibacterial agent, disinfectant, chelating agent or mixtures thereof; more preferably wherein the at least one excipient suitable for dermatological use is selected from the following list: water, ethanol, benzyl alcohol, diol molecule, urea, ammonium thioglycolate, thioanisole, tris(hydroxymethyl)aminomethane, phosphate buffer, sodium hydroxide, sodium chloride, citrate buffer, or a combination of two or more thereof.

13. Cosmetic composition according to any of the previous claims, for use as antimicrobial agent against infection; preferably against infections of *Trichophyton mentagrophytes* strain, and/or toenails infections.

14. Cosmetic composition according to any of the previous claims, for use in hair or scalp treatment.

15. A shampoo, lotion, serum, cream, conditioner, foam, elixir, oil, aerosol or mask comprising the cosmetic composition described in any of the previous claims.
